# EUROPEAN PATENT SPECIFICATION

(11) **EP 3 963 331 B1**
(45) Date of publication and mention of the grant of the patent: **19.06.2024**
(21) Application number: 20721642.5
(22) Date of filing: 01.05.2020
(51) Int. Cl.: G01N 33/574, G01N 33/68, G01N 33/72

(54) **METHOD FOR THE DETECTION OF CANCER**
VERFAHREN ZUR KREBSERKENNUNG
MÉTHODE DE DÉTECTION DU CANCER

(30) Priority: 02.05.2019 GB 201906199; 28.08.2019 US 201962893082 P
(43) Date of publication of application: 09.03.2022
(73) Proprietor: Belgian Volition SRL, 5032 Isnes (BE)
(72) Inventor: MICALLEF, Jacob Vincent, 5032 Isnes (BE); ECCLESTON, Mark Edward, 5032 Isnes (BE); HERZOG, Marielle, 5032 Isnes (BE); TERRELL, Jason Bradley, 5032 Isnes (BE)
(74) Representative: Sagittarius IP
(86) International application number: PCT/EP2020/062190
(87) International publication number: WO 2020/221921

(56) References cited:
- WO-A1-2016/067029
- WO-A1-2017/027379
- WO-A2-2009/090269
- CHARLOTTE THÅLIN ET AL: "Citrullinated histone H3 as a novel prognostic blood marker in patients with advanced cancer", PLOS ONE, vol. 13, no. 1, 11 January 2018 (2018-01-11), page e0191231, XP055656879, DOI: 10.1371/journal.pone.0191231
- PRIYA PATEL ET AL: "Markers of Inflammation and Infection in Sepsis and Disseminated Intravascular Coagulation", CLINICAL AND APPLIED THROMBOSIS/HEMOSTASIS, vol. 25, 17 January 2019 (2019-01-17), XP055656882, US ISSN: 1076-0296, DOI: 10.1177/1076029619843338
- TAO GU ET AL: "Impact of Elevated Circulating Histones on Systemic Inflammation after Radiofrequency Ablation in Lung Cancer Patients", BIOMED RESEARCH INTERNATIONAL, vol. 2017, 1 January 2017 (2017-01-01), pages 1-6, XP055656889, ISSN: 2314-6133, DOI: 10.1155/2017/6894832
- ELLA GRILZ ET AL: "Citrullinated histone H3, a biomarker for neutrophil extracellular trap formation, predicts the risk of mortality in patients with cancer", BRITISH JOURNAL OF HAEMATOLOGY, 9 April 2019 (2019-04-09), XP055656894, GB ISSN: 0007-1048, DOI: 10.1111/bjh.15906
- LOUISE RASMUSSEN ET AL: "Circulating cell-free nucleosomes as biomarkers for early detection of colorectal cancer", ONCOTARGET, vol. 9, no. 12, 13 February 2018 (2018-02-13), XP055656897, United States ISSN: 1949-2553, DOI: 10.18632/oncotarget.21908
- MONIKA BAUDEN ET AL: "Circulating nucleosomes as epigenetic biomarkers in pancreatic cancer", CLINICAL EPIGENETICS, vol. 7, no. 1, 1 December 2015 (2015-12-01), XP055656901, GB ISSN: 1868-7075, DOI: 10.1186/s13148-015-0139-4
- ERIN L. SYMONDS ET AL: "FIT for purpose: enhanced applications for faecal immunochemical tests", JOURNAL OF LABORATORY AND PRECISION MEDICINE, vol. 3, 1 March 2018 (2018-03-01), pages 28-28, XP055695684, DOI: 10.21037/jlpm.2018.03.03
- MASAKI YAMAGUCHI ET AL: "Plasma cytokine levels and the presence of colorectal cancer", PLOS ONE, vol. 14, no. 3, 18 March 2019 (2019-03-18) , page e0213602, XP055626666, DOI: 10.1371/journal.pone.0213602

## Description

### FIELD OF THE INVENTION

The invention relates to a body fluid test method for the detection of cancer using a combination biomarker panel comprising at least one cytokine molecule and at least one cell free chromatin fragment.

### BACKGROUND OF THE INVENTION

Cancer is a common disease with a high mortality. The biology of the disease is understood to involve a progression from a pre-cancerous state leading to stage I, II, III and eventually stage IV cancer. For the majority of cancer diseases, mortality varies greatly depending on whether the disease is detected at an early localized stage, when effective treatment options are available, or at a late stage when the disease may have spread within the organ affected or beyond when treatment is more difficult. Late stage cancer symptoms are varied including visible blood in the stool, blood in the urine, blood discharged with coughing, blood discharged from the vagina, unexplained weight loss, persistent unexplained lumps (e.g. in the breast), indigestion, difficulty in swallowing, changes to warts or moles as well as many other possible symptoms depending on the cancer type. However, most cancers diagnosed due to such symptoms will already be late stage and difficult to treat. Most cancers are symptomless at early stage or present with non-specific symptoms that do not help diagnosis. Cancer should ideally therefore be detected early using cancer tests.

The cancer with the highest mortality rate in developed countries is lung cancer. The five-year survival rate for lung cancer is >50% for cases detected when the disease is still localized within the lungs, but only 5% when the disease has spread to other organs. Unfortunately, most lung cancer cases are diagnosed when already metastatic (57%) whilst only 16% are diagnosed at an early stage. The 5-year survival rate for breast cancer is around 85% for those in whom the disease is detected at stage I, but only about 10% for those in whom stage IV metastatic disease is detected. Similarly, the 5-year survival rate for colorectal cancer (CRC) is >90% when detected at stage I, but only about 10% for those in whom stage IV metastatic disease is detected. Many other cancer diseases follow a similar pattern and, for this reason, many countries have screening programs to identify individuals with cancerous or precancerous conditions. The cancers most commonly screened for are breast cancer by mammography scanning, cervical cancer by cervical smear sample testing for HPV and/or inspection for abnormal cervical cells, colorectal cancer (CRC) by Fecal Immunochemical Testing (FIT) and/or colonoscopy and, more recently, lung cancer by low dose computed tomography (LDCT) scanning. Blood measurements of Prostate Specific Antigen (PSA), though not approved by the FDA as a screening test for prostate cancer, are also often performed on healthy men.

Some cases of cancer, for example cancers of the breast or testis, may be detected by palpation of the body for inappropriate lumps, nodules or masses. Any such lumps may or may not be cancerous in nature and further investigation may be required to determine whether a lump is malignant or benign in nature. However, palpation of internal organs such as the lungs, colon or pancreas is not possible and other cancer tests are necessary. Most cancer tests can be broadly categorized as either (i) a scan to visualize a nodule, mass or lump in the body, (ii) a tissue biopsy to search for abnormal cells in the target organ or (iii) a body fluid test for a substance released by the cancer or associated or surrounding tissues. All current cancer screening methods suffer from disadvantages. Scans allow visual detection of lumps or nodules but, like palpation, often fail to differentiate between malignant nodules and indolent or non-malignant (e.g. fibrous) lumps leading to poor specificity and/or overdiagnosis. Tissue biopsy involves highly invasive surgery or needle biopsies for most tissues (e.g. lung, liver, kidney, prostate). Even tissues that are relatively accessible (e.g. cervical tissue) require invasive and intrusive biopsy procedures. Blood and other body fluid tests are low cost and non-invasive but rare.

Cervical smear testing is a long established cancer screening method. It has proved effective in preventing disease in individual women and lowering disease prevalence in the population at large because, (i) it detects precancerous cervical tissue which can be removed before it develops into cancer, and (ii) cervical cancer affects younger women so prevention may preserve many quality life years for an individual. A cervical smear test involves taking a smear sample of cells from the surface of the cervix which is examined for the presence of any abnormal cancerous or precancerous cells. The smear may be examined either by cytological examination of the cells or by testing the cells for incorporation of Human Papilloma Virus (HPV) DNA. Cytological examination of the sample gives a correct result for 70%-80% of women tested and HPV testing of the sample for 90%-95% of women tested. However, cervical smear sampling is invasive and intrusive which affects patient uptake of the test. Compliance varies with women's age but is around 80% overall. There is no commonly used blood test for cervical cancer.

The primary screening method for CRC employed in the USA is colonoscopy. This test is accurate for CRC detection and also identifies most colorectal polyps or adenomas which are potential precancers that may develop into CRC. Removal of precancerous colorectal polyps leads to a favourable prognosis for the patient. However, colonoscopy is expensive and costs >$1000 in the USA. The test is also invasive and intrusive requiring a surgical admission and can occasionally cause injury (for example by tearing of the bowel). The procedure is usually performed under anaesthesia and requires an unpleasant preparation by the patient in advance in which the bowel is thoroughly flushed. Moreover, because the prevalence of CRC is low, the disease is detected in only approximately 0.5% of screening colonoscopies so the vast majority of people screened are subjected to a surgical procedure for little benefit. All these disadvantages affect the uptake of the test and compliance for CRC screening by colonoscopy in the USA is poor at around 60% of the screening age population. Because of its disadvantages, colonoscopy is not used as a frontline CRC detection or screening method in most countries of the world.

Some healthcare providers employ a related method called sigmoidoscopy in which a shorter scope is used to examine the descending colon only. Although this method misses two thirds of the colon, it does examine the area where cancers are most commonly observed. The disadvantages of sigmoidoscopy are similar to those of colonoscopy and it is not commonly used as a frontline test for similar reasons. Virtual colonoscopy, or computerized tomography (CT) colonography, is also used. This procedure employs a combination of x-rays and computer technology to create images of the rectum and colon to detect colorectal tumours and adenomas.

The most commonly used CRC detection and screening methods involve a two-stage procedure in which the population of screening age is first screened with a non-invasive frontline fecal test to identify a subgroup of the screening population in whom there is a higher risk of CRC. People who test positive in the fecal test are referred for a follow-up colonoscopy and about 5% of these people will typically be found to have CRC. The result of fecal screening is negative for a majority of people, so the two-stage method prevents unnecessary colonoscopies on most people with no lesion.

The principle underlying current fecal tests for CRC is the detection of bleeding into the colon or rectum. For example, when the colon or rectum is partially blocked by an intruding cancerous or precancerous growth, movement of the stool past the blockage is likely to cause injury and bleeding. This bleeding is detected by testing the fecal sample for the presence of hemoglobin. As the degree of bleeding may vary greatly from day to day, the test may need to be performed several times on separate days.

All current fecal CRC tests are designed to detect fecal hemoglobin. The guaiac fecal occult blood test (FOBT or gFOBT) is a chemical test method for hemoglobin in which the patient or operator typically smears a small amount of feces on to an alpha-guaiaconic acid coated paper or other substrate. If blood is present in the feces, addition of hydrogen peroxide to the paper produces a rapid colour change through the oxidation of alpha-guaiaconic acid to a blue coloured quinone in a reaction catalysed by heme (a component of hemoglobin). The consumption of meat (and hence heme) as well as some vegetables, that contain other catalyst molecules that behave like heme in the test, can cause false positive results. Similarly, some substances, including vitamin C can lead to false negative results so dietary restriction is often advised prior to the test. Guaiac FOBT tests can have high clinical specificity depending on the cut-off used and have 60-70% sensitivity for detection of CRC. Detection of precancerous adenomas is poor. Chemical FOBT methods were the method of choice in the past and, although still widely used, are being displaced by FIT methods.

FIT methods (also called iFOBT or FOBTi) are essentially immunoassay tests for human hemoglobin in fecal samples. FIT methods are less susceptible to false positive and negative results due to interference of dietary factors and can detect smaller amounts of blood in the feces. These tests detect around 72% of CRC cases at a specificity of 95% and so detect slightly more clinically relevant cancer lesions than gFOBT with a similar specificity. Detection of adenomas is poor. FIT and gFOBT tests are non-invasive and low cost but require manipulation of feces by the patient which is unpleasant to perform and leads to low compliance. Patient compliance with FIT and FOBT methods is 60%-70% in the most compliant European countries with national screening programmes but is as low as 10%-20% in many countries. Moreover, the tests are not completely reliable. FIT misses around 30% of CRC cases and in addition, the majority of FIT positive subjects do not have cancer and hence undergo a follow up invasive colonoscopy for little or no benefit. The Cologuard fecal test for CRC, produced by Exact Sciences, employs several fecal DNA measurements, in addition to a fecal hemoglobin measurement, to increase the accuracy of the test to a 92% sensitivity at a specificity of 87%. Blood tests for CRC detection are not used clinically primarily due to their lack of accuracy. For example, the only blood test currently approved by the FDA for CRC is the Epi Procolon test which detects 68% of CRC cases with a specificity of 80% (Potter *et al,* 2014).

Breast cancer screening by mammography is routinely carried out for the early detection of the disease when treatment regimens have better outcomes. Mammography uses low dose x-rays to visualize any small lump in the breast before it can be felt, or to show tiny clusters of calcium called microcalcifications. Histological confirmation of cancer by biopsy is necessary as lumps or specks may be due to other conditions like fatty cells or cysts, and up to 50% of positive findings are false positive results. In addition, some true positive results cause overdiagnosis where a small lump is indolent in nature and will not progress to a lifethreatening disease. False positives and overdiagnosis may lead to unnecessary invasive procedures and exposure to further x-rays. These disadvantages and the inherent danger of x-ray exposure lead to reduced patient compliance. Current blood tests for breast cancer detection are not sufficiently accurate for routine clinical use. For example CA15-3, the most commonly used tumour marker for breast cancer, detects 19% of breast cancer cases at 95% specificity (Wojtacki *et al,* 1994).

Lung cancer screening by LDCT has recently been recommended as a screening test for the early detection of the disease in high risk subjects, for example long term heavy smokers. LDCT uses low dose x-rays to visualize small early lumps or nodules in the lung. However, as with other scanning methods, any lumps observed may or may not be cancerous in nature and histological confirmation of cancer by biopsy is necessary. Up to 40% of positive findings on LDCT are false positive results where the lesion detected is not cancerous. In addition, many nodules of unknown aetiology are found where the nodule may or may not be malignant in nature, but is too small to biopsy. False positive results may lead to unnecessary invasive procedures and nodules of unknown aetiology may lead to repeated follow-up scans to monitor lumps with repeated exposure to further x-rays. Blood tests for lung cancer detection are not used clinically primarily due to their lack of accuracy. For example, a miRNA test that detects 21% of lung cancer cases at 76% specificity has been proposed as a front-line screening test and the EarlyCDT-Lung test that detects 41% of lung cancer cases at 87% specificity is under evaluation as a primary screening test (Midthun, 2016)

An important failing of current screening methods is low patient compliance because failure to undergo screening may lead to early death for the patient and increases the burden of expensive late stage cancer treatment for health providers. Compliance with colonoscopy in the USA for CRC screening is poor at approximately 60% of people over the age of 50 years. The remaining unscreened people are clearly at increased risk of CRC. Compliance with European FIT screening programmes for CRC is similarly poor at 60%-70%. Mammography and LDCT testing involve exposure to damaging x-rays and frequent or repeated testing has the potential to cause of cancer. At the time of writing, LDCT is a recent screening development but early experience indicates poor compliance, perhaps as low as 20%. The reasons for this may include the need for repeat scans every 3-6 months on nodules discovered with unknown aetiology, exposing subjects to repeated x-ray doses with the potential to accelerate cancer development in what may have been otherwise slow growing nodules. The United States Preventative Services Task Force (USPSTF) has identified a need for biomarkers to accurately discriminate between benign and malignant nodules identified on LDCT scanning (Moyer, 2014). All current cancer screening methods suffer from combinations of poor accuracy, overdiagnosis, high cost, high invasiveness, exposure to x-rays and poor patient compliance.

The majority of commonly occurring cancers are not screened for including, for example, lymphoma, kidney, bladder, pancreatic, uterus, myeloma, thyroid, ovarian or liver cancers. This is a reflection of the absence of good cancer blood tests for these diseases. Prostate cancer is an unusual case because, whilst no screening test is approved or recommended, the PSA test is often performed for healthy men and a positive result will lead to follow on tests for suspected prostate cancer. The main advantages of the PSA test do not stem from its accuracy but from its nature as a blood test. It is a low cost, non-invasive test that can be included in routine health checks that obviate most compliance issues because a hospital visit is not required, no special preparation is required by the patient and the small volume of blood required (<100µL) means that a dedicated blood draw is not necessary so the test can be included in a menu with other routine tests (*e.g*. for cholesterol, blood sugar and liver enzymes) requested by the physician as part of a routine health check.

To address the need for a simple routine cancer blood test, many blood borne biomarkers have been investigated as potential cancer tests including carcinoembryonic antigen (CEA) for CRC, alpha-fetoprotein (AFP) for liver cancer, CA125 for ovarian cancer, CA19-9 for pancreatic cancer, CA 15-3 for breast cancer and PSA for prostate cancer. However, their clinical accuracy is too low for routine diagnostic use and they are considered to be better used for patient monitoring.

More recently, hypermethylation of specific gene sequences have been investigated for use as diagnostic biomarkers for cancers in blood. For example, the DNA methylation status of specific genes or loci have been investigated by selective bisulphite deamination of cytosine, but not 5-methylcytosine, to uracil, leading to a primary DNA sequence change that can be detected by sequencing or other means (Yang *et al,* 2004). One such test for hypermethylation of the SEPTIN-9 gene is the only blood test currently approved for CRC detection by the Food and Drug Administration (FDA) in the USA. This test was found to detect 68% of CRC cases with a specificity of 80%. There is similarly a great deal of interest in the use of circulating tumour DNA (ctDNA) as the basis for cancer detection in a blood test. However, whilst ctDNA tests identify late-stage cancer, they do not detect early stage cancer. Moreover, ctDNA tests are expensive and require large volumes of blood. These disadvantages mean that it is unlikely to be used as a routine cancer screening method.

Workers in the field have also investigated numerous other biomarkers for the detection of cancer including circulating cell free nucleosomes *per se* (Holdenrieder *et al,* 2001) and inflammatory molecules such as TNFα, interleukin-6 (IL-6) and interleukin-8 (IL-8) (Chadha *et al,* 2014).

Although it is well known that circulating levels of cell free nucleosomes *per se* may be elevated in a variety of cancer conditions, cell free nucleosome measurements have not been used clinically to detect cancer or for any other clinical purpose (Holdenrieder *et al,* 2001). A major disadvantage of measurements of cell free nucleosome *per se* in clinical use is that elevated levels are a non-specific indicator of cell death and have been reported for a plethora of conditions including gynaecological diseases, autoimmune diseases, inflammatory diseases, stroke, cardiac disease, sepsis, graft vs host disease trauma and following burns, surgery or exercise (Holdenrieder *et al,* 2005 and Holdenrieder and Stieber, 2009). Thus, measurements of elevated levels of nucleosomes *per se* are considered too non-specific an indicator of disease to be used in oncology.

Circulating cell free nucleosomes containing particular epigenetic signals including particular post-translational modifications, histone isoforms, modified nucleotides and non-histone chromatin proteins have also been investigated as markers of cancer (as referenced in WO2005019826, WO2013030577, WO2013030579 and WO2013084002).

Circulating levels of cytokine inflammatory molecules including many of the interleukin proteins have also been reported to be altered in cancer. There are more than 50 interleukin and related proteins encoded in the human genome. These are denoted Interleukin 1 (IL-1), Interleukin 2 (IL-2) and so on. Elevated levels of many cytokines have been reported in cancer including, for example without limitation, IL-1, IL-2, IL-6, IL-7, IL-8, IL-11, IL-12, TNF-α and CRP (Lipitz and Harris, 2016 and Allin *et al,* 2011). For example, the role of IL-6 in tumorigenesis has been investigated in a wide range of human cancers including CRC as well as lymphoma, glioma, melanoma, breast, ovarian, renal and pancreatic cancer (Wang and Sun, 2014). Elevated circulating IL-6 levels are associated with tumorigenesis, disease progression and poor prognosis in many cancers including colorectal, prostate, skin, breast, lung, oesophageal, liver, pancreatic, gastric, gynaecological, kidney, bladder and haematological cancers (Taniguchi and Karin, 2014). Similarly, circulating IL-8 levels are increased in many cancers including colorectal, gastric, melanoma, ovarian, pancreatic, prostate and breast cancer.

However, cytokine markers are not used routinely as tests for cancer. A major disadvantage of these markers is that they are non-specific markers involved a large range of conditions including obesity, diabetes, autoimmune disorders, inflammatory diseases and infection.

WO2017/027379 and Thalin et al. (2018) describe methods for screening for the presence of cancer and several other indications in an individual comprising the steps of providing a plasma sample to be tested from the individual; and measuring the presence and/or quantity in the plasma sample of citrullinated histone H3 (H3Cit). Patel et al. (2019) describes markers of inflammation and infection in sepsis and disseminated intravascular coagulation. Gu et al. (2017) describes the changes of circulating histones following radiofrequency ablation (RFA) in lung cancer patients and their impact on systemic inflammation. WO2009/090269 describes methods for the prediction, prognosis and/or diagnosis of metastasis using a panel of proteins including histone H4 and IL-25. Symonds et al. (2018) reviewed enhanced applications of FIT with an aim to reduce the burden on limited colonoscopy resources. Yamaguchi et al. (2019) describes the possibility of plasma cytokines for the screening of colorectal cancer.

Despite recent advances, no blood test methods are used routinely for cancer screening with the possible exception of use of PSA for prostate cancer detection despite recommendations against this. There is a need to develop non-invasive blood tests for individual cancers as well as for cancer diagnosis in general for use as a general cancer screen, or to rule cancer in or out as a potential diagnosis in symptomatic patients or as an adjunct to other cancer detection methods.

### SUMMARY OF THE INVENTION

According to a first aspect of the invention, there is provided the *in vitro* use of a panel of biomarkers in a body fluid sample for diagnosing and/or monitoring a cancer, wherein the biomarkers comprise histone isoform H3.1 and Interleukin-6 (IL-6).

According to a further aspect of the invention, there is provided a method of diagnosing cancer in a patient, comprising:
detecting or measuring IL-6 and histone isoform H3.1, in a body fluid sample obtained from the patient; and
using the level or concentration of IL-6 and histone isoform H3.1 detected in the body fluid sample to determine if the patient has cancer.

According to a further aspect of the invention, there is provided a kit comprising reagents to detect IL-6 and histone isoform H3.1 in a body fluid sample.

According to a further aspect of the invention, there is provided a method for assessing the suitability of a patient for a colonoscopy, comprising:
(i) detecting or measuring the level of fecal haemoglobin in a fecal sample obtained from the patient;
(ii) detecting or measuring the level of IL-6, and the level of histone isoform H3.1, in a body fluid sample obtained from the patient; and
(iii) using the measured fecal haemoglobin, IL-6 and histone isoform H3.1 level, as an indicator of the suitability of the patient for colonoscopy.

### BRIEF DESCRIPTION OF THE FIGURES

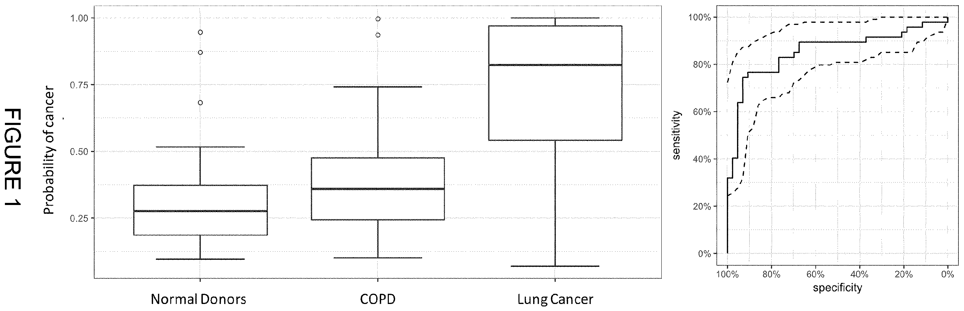

**Figure 1****:** Box-plot and Receiver Operating Characteristic (ROC) curve for a two-assay panel comprising nucleosomes containing histone H3.1 and IL-6 trained to optimize discrimination between patients with lung cancer compared to normal donors in a 144 subject lung cancer cohort. The box-plot is expressed as the probability of cancer for any given subject calculated by logistic regression analysis.

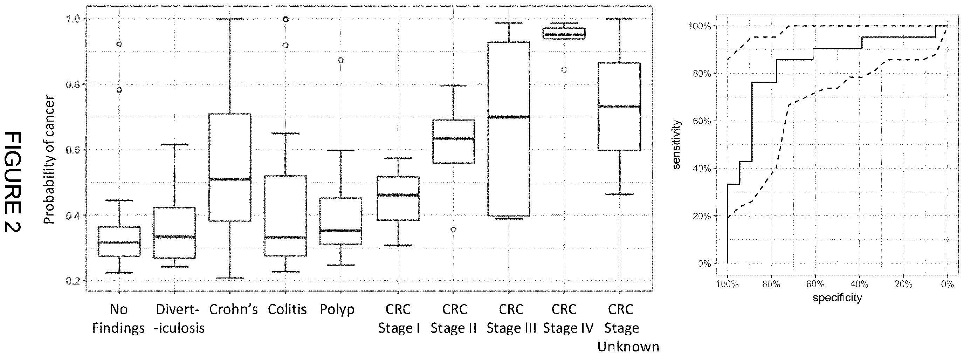

**Figure 2****:** Box-plot and ROC curve for a three-assay panel comprising measurements of nucleosomes *per se,* nucleosomes containing histone isoform H3.1 and IL-6 trained to optimize discrimination between patients with colorectal cancer (CRC) compared to patients with symptoms but no findings on colonoscopy in a 100 subject CRC cohort. The box-plot is expressed as the probability of cancer for any given subject calculated by logistic regression analysis.

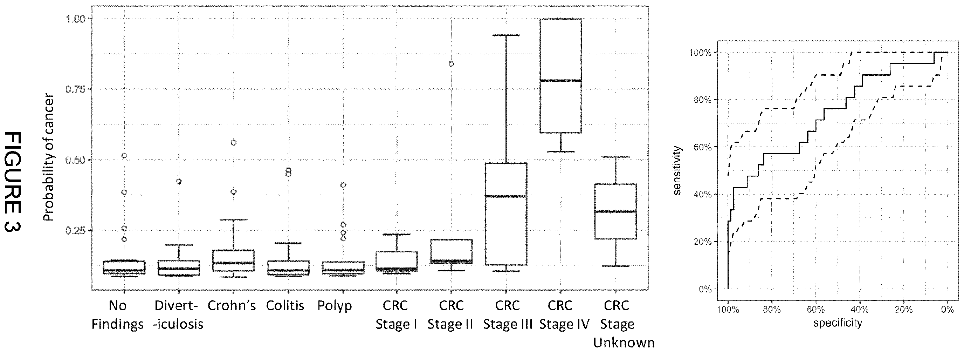

**Figure 3****:** Box-plot and ROC curve for a three-assay panel comprising measurements of nucleosomes *per se,* nucleosomes containing histone isoform H3.1 and IL-6 trained to optimize discrimination between patients with CRC compared to both subjects with no findings on colonoscopy and patients with non-malignant benign colon or bowel disease in a 100 subject CRC cohort. The box-plot is expressed as the probability of cancer for any given subject calculated by logistic regression analysis.

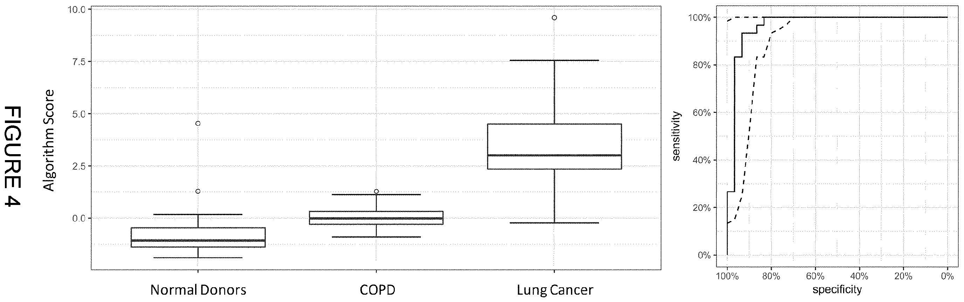

**Figure 4****:** Box-plot and ROC curve for a two-assay panel model (the lung/CRC combined model) comprising nucleosomes containing histone isoform H3.1 and IL-6 trained to optimize discrimination between patients with CRC or lung cancer compared to both normal donors and subjects with no findings on colonoscopy applied to a 70 subject validation cohort including 30 lung cancer patients, 30 normal donor subjects and 10 patients with chronic obstructive pulmonary disease (COPD). The box-plot is expressed as the model/algorithm output for each subject calculated by logistic regression analysis.

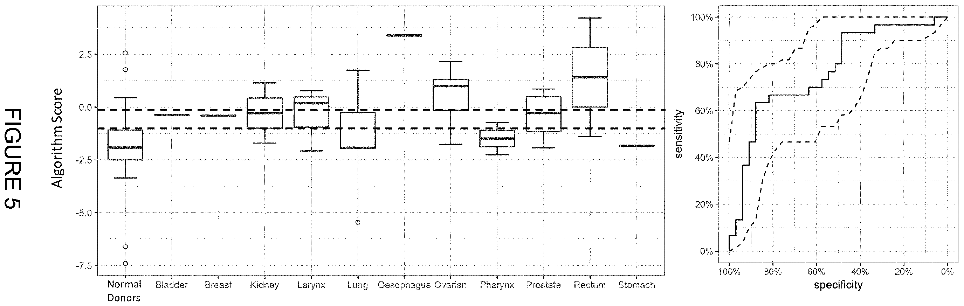

**Figure 5****:** Box-plot and ROC curve for a two-assay panel model (the lung/CRC combined model) comprising nucleosomes containing histone isoform H3.1 and IL-6 trained to optimize discrimination between patients with CRC or lung cancer compared to both normal donors and subjects with no findings on colonoscopy applied to a 63 subject validation cohort including 30 patients with a variety of cancer diseases and 33 normal donor subjects. The box-plot is expressed as the model/algorithm output for each subject calculated by logistic regression analysis. The upper and lower dashed lines indicate the cut-off values at 90% specificity and 80% specificity respectively.

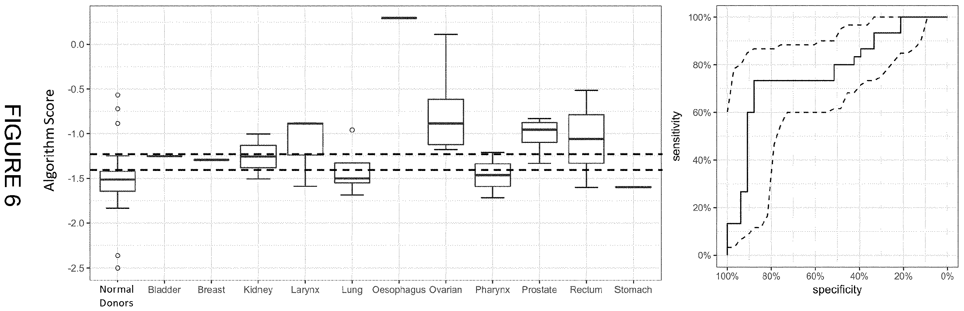

**Figure 6****:** Box-plot and ROC curve for a two-assay panel comprising nucleosomes containing histone isoform H3.1 and IL-6 trained on a 100 subject CRC cohort to optimize discrimination between patients with CRC compared to patients with symptoms but no findings on colonoscopy, applied to a 63 subject validation cohort including 30 patients with a variety of cancer diseases and 33 normal donor subjects. The box-plot is expressed as the model/algorithm output for each subject calculated by logistic regression analysis. The upper and lower dashed lines indicate the cut-off values at 90% specificity and 80% specificity respectively.

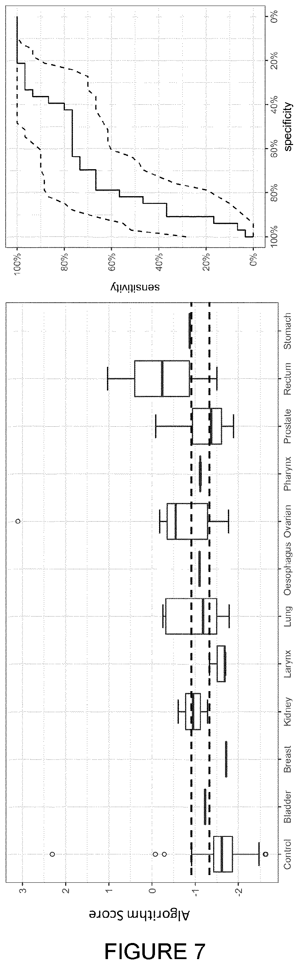

**Figure 7****:** Box-plot and ROC curve for a three-assay panel comprising nucleosomes *per* se, nucleosomes containing histone isoform H3.1 and IL-6 trained on a 100 subject CRC cohort to optimize discrimination between patients with CRC compared to patients with symptoms but no findings on colonoscopy, applied to a 63 subject validation cohort including 30 patients with a variety of cancer diseases and 33 normal donor subjects. The box-plot is expressed as the model/algorithm output for each subject calculated by logistic regression analysis. The upper and lower dashed lines indicate the cut-off values at 90% specificity and 80% specificity respectively.

### DETAILED DESCRIPTION OF THE INVENTION

According to a first aspect of the invention, there is provided the *in vitro* use of a panel of biomarkers in a body fluid sample for diagnosing and/or monitoring a cancer, wherein the biomarkers comprise histone isoform H3.1 and Interleukin-6 (IL-6).

In the Examples provided herein, we have shown that there is an increase in blood levels of several interleukins in cancer patients over levels observed in people with no cancer. We have shown this for a variety of cancer diseases. We have also shown that there is an increase in circulating levels of nucleosomes *per se* in cancer patients over levels observed in people with no cancer. We have also shown that there is an increase in circulating levels of nucleosomes containing a particular histone isoform in cancer patients over levels observed in people with no cancer. We have also shown that the measurement of both interleukin molecules and nucleosome moieties as combined biomarker panel is highly accurate for the detection of cancer diseases, both in terms of the proportion of patients with cancer and the breadth of cancer disease types that can be detected.

It will be clear to those skilled in the art that methods of the invention will detect all or the majority of cancer types tested and all of the common cancer types. As shown in the Examples, the 2-assay panel and 3-assay panel embodiments exemplified detect most cancer cases and all or most types of cancer. It will also be clear to those skilled in the art that further nucleosome and/or cytokine assays may be added to such panels to further increase either or both of the sensitivity of detection and the breadth of cancers detected. In other embodiments, other non-nucleosome or non-cytokine assays may be added to such panels to increase their performance. For example, without limitation, CA19-9 may be added to increase the performance for pancreatic cancer, CEA for CRC, AFP for liver cancer, CA125 for ovarian cancer, PSA for prostate cancer and so on.

We have shown that combinations of assays including cytokine assays and nucleosome assays produced improved results for the discrimination of cancer patients from normal (healthy) subjects, both in terms of the accuracy of cancer detection and in terms of the variety of cancer diseases detected. We have also shown that such panel of assays are effective where the nucleosome assay is for nucleosomes *per* se or for nucleosomes containing a histone isoform or variant. We now report the development of non-invasive blood tests that predict the presence of cancer in a subject.

The term "chromatin fragment" as used herein refers to a complex of proteins and nucleic acid whose origin lies in the chromosome of a cell. A fragment of chromatin may contain a nucleosome and/or associated DNA and/or any of a variety of non-histone chromatin associated proteins in a multi-protein-nucleic acid complex. Some examples of non-histone chromatin associated proteins (i.e. protein adducts) include transcription factors, cofactors, co-activators, co-repressors, RNA polymerase moieties, elongation factors, chromatin remodelling factors, mediators, STAT moieties, upstream binding factor (UBF) and others.

The nucleosome is the basic unit of chromatin structure and consists of a protein complex of eight highly conserved core histones (comprising of a pair of each of the histones H2A, H2B, H3, and H4). Around this complex is wrapped approximately 146 base pairs of DNA. Another histone, H1 or H5, acts as a linker and is involved in chromatin compaction. The DNA is wound around consecutive nucleosomes in a structure often said to resemble "beads on a string" and this forms the basic structure of open or euchromatin. In compacted or heterochromatin this string is coiled and super coiled into a closed and complex structure (Herranz and Esteller (2007)).

References to "nucleosome" may refer to "cell free nucleosome" when detected in body fluid samples. It will be appreciated that the term "cell free nucleosome" used throughout this document is intended to include any cell free chromatin fragment that includes one or more nucleosomes. Epigenetic signal structures/features of a cell free nucleosome as referred herein may comprise, without limitation, one or more histone post-translational modifications, histone isoforms/variants, modified nucleotides and/or proteins bound to a nucleosome as a nucleosome-protein adduct.

The term "component thereof" as used herein refers to a part of the nucleosome, *i.e.* the whole nucleosome does not need to be detected. A component of the cell free nucleosomes may be selected from the group consisting of: a histone protein (*i.e.* histone H1, H2A,
H2B, H3 or H4), a histone post-translational modification, a histone variant or isoform, a protein bound to the nucleosome (*i.e*. a nucleosome-protein adduct), a DNA fragment associated with the nucleosome and/or a modified nucleotide associated with the nucleosome. The *in vitro* use comprises nucleosomes containing histone H3.1.

References to "nucleosomes *per* se" refers to the total nucleosome level or concentration present in the sample, regardless of any epigenetic features the nucleosomes may or may not include. This type of assay is also often referred to as a total nucleosome assay and typically involves detecting a histone protein common to all nucleosomes, such as histone H4 or histone H3. As described herein, histone proteins form structural units known as nucleosomes which are used to package DNA in eukaryotic cells. In one embodiment, the histone protein is a core histone, such as H2A, H2B, H3 or H4. As previously reported in WO2016067029, particular histone variants, such as histone H3.1, H3.2 or H3t, may be used to isolate cell free nucleosomes originating from tumour cells. Therefore, the level of cell free nucleosomes of tumour origin may be detected.

Total cell free nucleosomes or nucleosomes *per se* may also be measured by quantifying their DNA fragment content. Circulating cell free DNA (ccfDNA) in blood comprises DNA fragments of <200 base pairs in length that circulate in the form of chromatin fragments and particularly nucleosomes. It has been shown that blood measurements of ccfDNA made using the PicoGreen nucleic acid stain method correlate 95% with ELISA measurements of cell free nucleosomes (Bjorkman *et al*; 2003). Thus, ccfDNA measurements can be considered equivalent to, or a proxy for, measurements of total nucleosome or total chromatin fragment levels. Typical methods, without limitation, for the quantification of ccfDNA as a proxy measurement of nucleosomes include quantification using a nucleic acid stain (for example PicoGreen, SYBR Green, SYBER Gold, Oxazole yellow and Thiazole Orange) or by Polymerase Chain Reaction (PCR) methods for the amplification and measurement of a repetitive DNA sequence of a single copy gene sequence or other methods.

Normal cell turnover in adult humans involves the creation by cell division of some 10¹¹ cells daily and the death of a similar number, mainly by apoptosis. During the process of apoptosis chromatin is broken down into mononucleosomes and oligonucleosomes which are released from the cells. Under normal conditions the levels of circulating nucleosomes found in healthy subjects is reported to be low. Elevated levels are found in subjects with a variety of conditions including many cancers, auto-immune diseases, inflammatory conditions, stroke and myocardial infarction (Holdenrieder and Stieber, 2009).

Mononucleosomes and oligonucleosomes can be detected by Enzyme-Linked ImmunoSorbant Assay (ELISA) and several methods have been reported (Salgame *et al,* 1997; Holdenrieder *et al,* 2001; van Nieuwenhuijze *et al,* 2003; WO2005019826; WO2013030577; WO2013030579; and WO2013084002). These assays typically employ an anti-histone antibody (for example anti-H2B, anti-H3 or anti-H1, H2A, H2B, H3 and H4) as capture antibody and detection antibody (which varies depending upon the moiety to be detected) or an anti-histone antibody as capture antibody and an anti-DNA antibody as detection antibody.

Circulating nucleosomes are not a homogeneous group of protein-nucleic acid complexes. Rather, they are a heterogeneous group of chromatin fragments originating from the digestion of chromatin on cell death and include an immense variety of epigenetic structures including particular histone isoforms (or variants), post-translational histone modifications, nucleotides or modified nucleotides, and protein adducts. It will be clear to those skilled in the art that an elevation in nucleosome levels will be associated with elevations in some circulating nucleosome subsets containing particular epigenetic signals including nucleosomes comprising particular histone isoforms (or variants), comprising particular post-translational histone modifications, comprising particular nucleotides or modified nucleotides and comprising particular protein adducts. Assays for these types of chromatin fragments are known in the art (for example, see WO2005019826, WO2013030579, WO2013030578, WO2013084002).

It will be understood that the terms "epigenetic signal structure" and "epigenetic feature" are used interchangeably herein. They refer to particular features of the nucleosome that may be detected.

The nucleosome moiety measured as part of the biomarker panel may be a circulating cell free nucleosome containing one or more particular or specified histone isoforms. Many histone isoforms are known in the art. The nucleotide sequences of a large number of histone isoforms are publicly available for example in the National Human Genome Research Institute NHGRI Histone DataBase (Mariño-Ramirez, L., Levine, K.M., Morales, M., Zhang, S., Moreland, R.T., Baxevanis, A.D., and Landsman, D. The Histone Database: an integrated resource for histones and histone fold-containing proteins. Database Vol.2011), the GenBank (NIH genetic sequence) DataBase, the EMBL Nucleotide Sequence Database and the DNA Data Bank of Japan (DDBJ).

The structure of nucleosomes can vary by post translational modification (PTM) of histone proteins. PTM of histone proteins typically occurs on the tails of the core histones and common modifications include acetylation, methylation or ubiquitination of lysine residues as well as methylation of arginine residues and phosphorylation of serine residues and many others. Many histone modifications are known in the art and the number is increasing as new modifications are identified (Zhao and Garcia, 2015).

In addition to the epigenetic signalling mediated by nucleosome histone isoform and histone post-translational modification composition, nucleosomes also differ in their nucleotide and modified nucleotide composition. Global DNA hypomethylation is a hallmark of cancer cells and some nucleosomes may comprise more 5-methylcytosine residues (or 5-hydroxymethylcytosine residues or other nucleotides or modified nucleotides) than other nucleosomes. 5-hydroxymethylation may be detected, for example, at CpG islands in the genome. In one embodiment, the DNA modification is selected from 5-methylcytosine or 5-hydroxymethylcytosine.

Non-histone proteins adducted to the nucleosome or chromatin fragment may include any protein that contains or includes a DNA binding domain or a nucleosome binding domain or a histone binding domain. Examples include transcriptions factors, structural chromatin proteins, CpG methyl-CpG binding domain proteins, high mobility group box proteins (e.g. HMGB1), epigenetic enzymes such as histone acetyl transferases, histone methyl transferases, histone deacetylases, DNA methyltransferases, PARP (poly-ADP ribose polymerase) binders and many others.

References to "transcription factor" refer to proteins that bind to DNA and regulate gene expression by promoting (*i.e*. activators) or suppressing (*i.e.* repressors) transcription. Transcription factors contain one or more DNA-binding domains (DBDs), which attach to specific sequences of DNA adjacent to the genes that they regulate.

All of the circulating nucleosomes and nucleosome moieties, types or subgroups described herein may be useful in the present invention.

Interleukins (ILs) are a group of cytokines, usually secreted by leukocytes, that act as signal molecules. They have key roles in stimulating immune responses and inflammation. They were first identified in the 1970s and have been designated numerically as more interleukin types have been discovered. Examples of interleukins include, but are not limited to: IL-1, IL-2, IL-3, IL-4, IL-5, IL-6, IL-7, IL-8, IL-9, IL-10, IL-11, IL-12, IL-13, IL-14 and IL-15.

According to the invention, the biomarker comprises Interleukin-6. Interleukin-6 (IL-6) is a cytokine with a wide variety of biological functions. It is a potent inducer of fever and the acute phase response. The sequence of human IL-6 is known in the art and is described at UniProt Accession No. P05231.

In one embodiment, the *in vitro* use additionally comprises Interleukin-8 as a biomarker. Interleukin-8 (IL-8, also known as CXCL8) is a chemokine that attracts immune cells, such as neutrophils, basophils, and T-cells. It is released from several cell types in response to an inflammatory stimulus. The sequence of human IL-8 is known in the art and is described at UniProt Accession No. P10145.

In one embodiment, the *in vitro* use additionally comprises Interleukin-10 as a biomarker. Interleukin-10 (IL-10) is an anti-inflammatory cytokine with a wide variety of biological functions. The sequence of human IL-10 is known in the art and is described at UniProt Accession No. P22301.

According to the invention, the at least one cell free chromatin fragment comprises histone isoform H3.1 and IL-6. In a further embodiment, the biomarkers comprise histone isoform H3.1, nucleosomes *per se* (*i.e.* the total level of cell free nucleosomes in the sample) and IL-6. In an alternative embodiment, the biomarkers consist of histone isoform H3.1, IL-6 and optionally nucleosomes *per se.* As described hereinbefore, there are multiple methods for detecting the level of nucleosomes *per se,* for example by using a reagent to detect a core histone protein, such as histone H3.

It will be clear to those skilled in the art that additional biomarkers (additional to nucleosome moieties and cytokine moieties) may be used in a biomarker panel to detect cancer and/or to identify the organ affected by the disease. In one embodiment, the *in vitro* use additionally comprises one or more biomarkers selected from: ferritin, Carcinoembryonic Antigen (CEA), CYFRA 21-1 (Cytokeratin 19 fragment), Cancer Antigen 125 (CA 125), Carbohydrate Antigen 19-9 (CA 19-9), Carbohydrate Antigen 15-3 (CA 15-3), Alpha FetoProtein (AFP), Prolactin, Human Chorionic Gonadotropin (HCG), Prostate Specific Antigen (PSA) and C-Reactive Protein (CRP).

The sample may be any biological fluid (or body fluid) sample taken from a subject including, without limitation, cerebrospinal fluid (CSF), whole blood, blood serum, plasma, menstrual blood, endometrial fluid, urine, saliva, or other bodily fluid (stool, tear fluid, synovial fluid, sputum), breath, *e.g.* as condensed breath, or an extract or purification therefrom, or dilution thereof. Biological samples also include specimens from a live subject, or taken post-mortem. The samples can be prepared, for example where appropriate diluted or concentrated, and stored in the usual manner. It will be understood that methods and *in vitro* uses of the present invention find particular use in blood, serum or plasma samples obtained from a patient. In one embodiment, the sample is a blood or plasma sample. In a further embodiment, the sample is a serum sample. In a further embodiment both serum and plasma samples are used for the measurement of different members of an assay panel.

In one embodiment, the biomarkers are for use in diagnosing the stage of cancer. Cancer may be assigned as stage I, stage II, stage III and stage IV. Stage definition varies with different cancer diseases and is known in the art. Typically, stage I is classified as when the cancer is small and confined locally to the tissue of origin. Stage II is classified as when the cancer has grown larger and beyond its origin into nearby tissues within the organ or to nearby lymph nodes. Stage III is classified as when the cancer has grown into nearby tissues beyond the organ of origin but has not spread to other more distant parts of the body. Stage IV is classified as when the cancer has spread to one or more distant parts of the body, such as the liver or lungs.

In one embodiment, the cancer is a stage I (for example stage IA or stage IB), stage II (for example stage IIA or stage IIB), stage III (for example stage IIIA, stage IIIB or stage IIIC) or stage IV (for example stage IVA or stage IVB) cancer. The invention may find utility in detecting early stage cancers, in particular stages I and II. Therefore, in one embodiment the cancer is a stage I, II or III. In a further embodiment, the cancer is stage I or stage II. In an alternative embodiment, the cancer is stage II or stage III. The invention may also find utility in detecting late stage cancers, in particular stages III and IV. Therefore, in one embodiment the cancer is a stage III or IV. In a further embodiment, the cancer is stage IV.

In one embodiment, the cancer is selected from: lung, colon, rectum, stomach, kidney, skin, prostate, cervix, breast, pharynx, larynx, ovary, oesophagus, oral, pancreas and bladder cancer. In a further embodiment, the cancer is selected from lung, colorectal, ovarian and prostate cancer, in particular lung and colorectal cancer. In a yet further embodiment, the cancer is lung cancer (such as non-small cell lung cancer or small cell lung cancer).

### Diagnosis methods

According to a further aspect of the invention, there is provided a method of diagnosing cancer in a patient, comprising:
detecting or measuring IL-6 and histone isoform H3.1, in a body fluid sample obtained from the patient; and
using the level or concentration of IL-6 and histone isoform H3.1 to determine if the patient has cancer.

In another embodiment, the method of the invention is performed to identify a subject at high risk of having a cancer and therefore in need of further testing (i.e. further cancer investigations), in particular to identify the organ location of a cancer. The further testing may involve one or more endoscopic or scanning methods including for example whole body scanning, MRI scanning, ultrasound scanning, LDCT, mammography, computerized tomography (CT) colonography or other scanning methods.

Therefore, according to a further aspect of the invention, there is provided a method for detecting a cancer and investigating the organ affected by the cancer, comprising:
detecting or measuring the level of IL-6 and histone isoform H3.1, in a body fluid sample obtained from the patient;
using the measured IL-6 and histone isoform H3.1 levels as an indicator of the presence of cancer in the body; and
detecting the location of said cancer (or tumour) in the body by an endoscopic or scanning method.

In addition to their uses as stand-alone tests, cancer rule-in or rule-out blood tests may be useful as adjunct methods to other screening modalities including, for example, in LDCT positive, mammography positive, PSA positive or FIT positive, persons. All of these tests are non-specific and therefore may be enhanced when used in conjunction with the method described herein. LDCT positive patients have a lump or nodule in their lung but the nodule may not be malignant and LDCT has a specificity of around 60%. Similarly, mammography positive patients have a lump or nodule in their breast but again the nodule may not be malignant. The specificity of the PSA test is also low at around 60-70%. In the case of asymptomatic subjects screened by FIT, only about 5% of persons found to be positive for fecal hemoglobin on screening are actually found to have CRC on follow-up colonoscopy. Many screening colonoscopies performed are thus, with hindsight, unnecessary.

In addition to screening colonoscopies, large numbers of monitoring or surveillance colonoscopies are also performed on patients who have previously been diagnosed with CRC or precancerous adenomas, which may have been treated or surgically removed, to monitor for any disease relapse or progression. FIT tests may also be used for monitoring or surveillance to select subjects for colonoscopy. Colonoscopies are also performed to investigate patients presenting with symptoms consistent with a possible CRC. Again, FIT tests may be used to select subjects for colonoscopy. In all these cases the majority of colonoscopies performed do not find a cancer and have a number of detrimental consequences for the patient and for healthcare providers including; (i) large numbers of unnecessary invasive medical colonoscopy procedures performed on persons with no colon lesions, (ii) large health care expenditure on expensive and unnecessary colonoscopies, (iii) due to historical insufficient investment in colonoscopy infrastructure, the colonoscopy capability of healthcare providers (particularly in European CRC screening programmes) is currently insufficient to meet medical demand resulting in an increasing backlog of unperformed colonoscopies and increased waiting times for colonoscopies for FIT positive persons, and (iv) this increased waiting time has resulted in potentially fatal delayed commencement of CRC treatment for those patients who do have CRC. A rule-in or rule-out blood test would overcome most of these problems by identifying those FIT positive persons whose colorectal bleeding is most probably due to cancer to triage those FIT positive persons most in need of urgent referral for colonoscopy. Similarly, subjects with a potentially cancerous nodule or other lesion of the lung or breast, identified by LDCT or mammography, could be tested using a rule-in or rule-out blood test to triage those patients whose lesions are most probably malignant in nature. This would avoid unnecessary biopsies and potentially dangerous repeated exposure to x-ray radiation. Men with elevated PSA levels could be tested using a rule-in or rule-out blood test to triage those patients in whom the cause of the elevated PSA level is most probably malignant in nature. Again, this may avoid some unnecessary repeat biopsies in men under active surveillance for prostate disease. Therefore, in one embodiment, the patient tested using a method of the invention is FIT positive, LDCT positive, mammography positive or PSA positive.

We have shown that use of circulating nucleosome levels and/or cytokine levels together with the numerical FIT score can be used to identify FIT positive subjects in whom no lesions are found on colonoscopy (i.e. true negatives). Therefore, the invention may be used to determine if a patient who has tested positive for fecal haemoglobin does not have a malignant colorectal lesion (i.e. the patient does not have cancer). Thus, the invention may be used to assess the suitability of patient for a colonoscopy.

According to a further aspect of the invention, there is provided a method for assessing the suitability of a patient for a colonoscopy, comprising:
(i) detecting or measuring the level of fecal haemoglobin in a fecal sample obtained from the patient;
(ii) detecting or measuring the level of IL-6 and the level of histone isoform H3.1, in a body fluid sample obtained from the patient; and
(iii) using the measured fecal haemoglobin, IL-6 and histone isoform H3.1 level, as an indicator of the suitability of the patient for colonoscopy.

Fecal haemoglobin tests are well known in the art. It will be understood that this aspect of the invention may be used in combination with patients who have already tested positive for fecal haemoglobin, i.e. the level of fecal haemoglobin has already been measured. Therefore, in one embodiment, step (i) may refer to: identifying patients who have been tested positive for fecal haemoglobin. In one embodiment, a patient is deemed to have tested positive for fecal haemoglobin if they have a fecal haemoglobin level which is greater than about 20µg haemoglobin/g feces (equivalent to 100 ng/ml in the diluted sample used in the OC-Sensor FIT test).

In one embodiment, the method additionally comprises measurement of the level of CRP in a body fluid sample obtained from the patient (i.e. circulating levels of CRP). In one embodiment, levels of fecal haemoglobin and levels of circulating CRP, and optionally also one or more nucleosome moieties and/or one or more interleukin levels, are used as an indicator of the absence of cancer in the body.

In one embodiment, the method additionally comprises measurement of one or more tumour markers to investigate the organ location of a cancer. Such tumour markers include CEA (suggestive of CRC or lung or pancreatic cancer), CYFRA 21-1 (suggestive of CRC), CA 125 (suggestive of ovarian cancer), CA 19-9 (suggestive of pancreatic cancer), CA 15-3 (suggestive of breast cancer), AFP (suggestive of liver cancer), Prolactin (suggestive of pituitary tumours), HCG (suggestive of ovarian cancer), PSA (suggestive of prostate cancer).

Therefore, according to a further aspect of the invention, there is provided a method for detecting a cancer and investigating the organ affected by the cancer, comprising:
detecting or measuring the level of IL-6 and histone isoform H3.1, in a body fluid sample obtained from the patient;
using the measured IL-6 and histone isoform H3.1 levels as an indicator of the presence of cancer in the body; and
detecting or measuring the level of one or more tumour markers to determine the location of the cancer.

It will be understood that detecting or measuring the level of a tumour marker is only required if the patient is first determined/indicated to have cancer (i.e. following the detection of the biomarker panel described herein).

In a further embodiment, a circulating tumour (ctDNA) or a cell free DNA (cfDNA) measurement is made in addition to or in place of a tumour marker measurement. Analysis of the ctDNA or cfDNA can inform on the site of a tumour, for example by methylated DNA sequencing or analysis, mutation sequencing or analysis, or nucleosome occupancy pattern sequencing or analysis. Therefore, in one embodiment, the method comprises analysing cfDNA or ctDNA associated with the cell free chromatin fragment to determine the location of the cancer.

Therefore, according to a further aspect of the invention, there is provided a method for detecting a cancer and investigating the organ affected by the cancer, comprising:
detecting or measuring the level of IL-6 and histone isoform H3.1, in a body fluid sample obtained from the patient;
using the measured IL-6 and histone isoform H3.1 levels as an indicator of the presence of cancer in the body; and
analysing cfDNA or ctDNA in the body fluid sample to detect the location of the cancer.

Again, it will be understood that analysing cfDNA or ctDNA is only required if the patient is first determined/indicated to have cancer (i.e. following the detection of the biomarker panel described herein).

In one embodiment, the method additionally comprises determining at least one clinical parameter for the patient. This parameter can be used in the interpretation of results. Clinical parameters may include any relevant clinical information for example, without limitation, gender, weight, Body Mass Index (BMI), smoking status and dietary habits. Therefore, in one embodiment, the clinical parameter is selected from the group consisting of: age, sex and body mass index (BMI). In one embodiment, the method is only used on patients greater than an age-dependent cut-off, such as patients older than 50 years old.

In one embodiment, a higher level of IL-6 and/or a higher level of the histone isoform H3.1 compared to the control is indicative of the presence and/or progression of cancer.

Data obtained by methods of the invention can be analysed using appropriate algorithms, for example those listed in **Table 1.**

**Table 1. Example models or algorithms for interpretation of the results of assay panels**

| |
|---|
| Panel score = a[IL-6] + b[H3.1-nucleosomes] |
| Panel score = a[IL-6] + b[nucleosomes *per se*] |
| Panel score = a[IL-6] + b[nucleosomes *per se*] + c[H3.1-nucleosomes] |

In one embodiment, the measuring step comprises the use of an algorithm listed in **Table 1.** Methods for deriving models or algorithms such as those in **Table 1** are well known in the art and suitable software packages are available. Typical software tools for this purpose include SPSS (Statistical Package for the Social Sciences) and "R". These software packages provide for linear and non-linear data modelling of clinical data.

Other methods of analysis of the results may also be used for the method of the invention. In one embodiment artificial intelligence models are used. In one embodiment individual assay cut-off levels are used and the patient is considered positive in the panel test if individual panel assay results are above (or below if applicable) the assay cut-off level for all or a minimum number of the panel assays (for example, one of two, two of two, two of three etc). In one embodiment of the invention a decision tree model or algorithm is employed for analysis of the results.

It will be clear to those skilled in the art, that any combination of the biomarkers disclosed herein may be used in panels and algorithms for the detection of cancer and that further markers may be added to a panel including these markers.

It will be clear to those skilled in the art that test sensitivity for patients with a cancer or a pre-cancer may be further improved by nucleosome assays and cytokine assays as part of a larger (blood) panel of assays to test for cancer.

In a preferred embodiment, the panel detects the level or concentration of IL-6 and the level or concentration of nucleosomes containing histone isoform H3.1.

### Differential diagnosis methods

A further advantage of the biomarkers of the invention is that they may be used in methods of differential diagnosis. Therefore, according to a further aspect of the invention, there is provided a method of differential diagnosis for a patient with suspected cancer, comprising:
(i) detecting or measuring the level of IL-6 and at histone isoform H3.1, in a body fluid sample obtained from the patient; and
(ii) comparing the levels obtained in step (i) to the level of IL-6 and histone isoform H3.1 in a body fluid sample obtained from a patient with a non-cancerous disease,
wherein a difference in the levels compared in step (ii) is indicative that the patient has cancer.

References to "non-cancerous disease" refer to diseases which are not cancerous, *e.g*. do not result in the development of a malignant tumour. They may sometimes be referred to as "benign" disease. The invention finds particular use in differential diagnosis methods where the suspected cancer and non-cancerous disease it is compared to are located in the same organ and/or present with similar symptoms. For example, differential diagnosis of suspected lung cancer compared to a non-cancerous lung disease. Non-cancerous lung diseases include, but are not limited to: asthma, bronchitis, chronic cough, chronic obstructive pulmonary disease (COPD), cryptococcosis, pneumonia, sarcoidosis and tuberculosis. The invention has particular use in diagnosing patients with suspected lung cancer (*e.g*. due to symptoms and/or identification of lumps in the lung) because the test differentiates between patients with lung cancer and other non-cancerous lung diseases. Therefore, in one embodiment, the non-cancerous lung disease is a disease which has similar signs and/or symptoms to lung cancer, such as COPD.

As shown in the Examples presented herein, biomarker panels of the invention were able to distinguish between patients with lung cancer (both small cell and non-small cell lung cancer) and patients with COPD and healthy patients (see **Figures 1** and **4**)**.**

As a further example, differential diagnosis may be conducted on patients with suspected colorectal cancer compared to a non-cancerous colon or bowel disease. Non-cancerous colon and bowel diseases include, but are not limited to: polyps, Crohn's disease, colitis, inflammatory bowel disease, ulcerative colitis and diverticulosis. The invention has particular use in diagnosing patients with suspected colorectal cancer (*e.g*. due to symptoms and/or identification of bleeding in the stool) because the test differentiates between patients with CRC and other non-cancerous diseases. Therefore, in one embodiment, the non-cancerous colon or bowel disease is a disease which has similar signs and/or symptoms to colorectal cancer, such as diverticulosis.

As shown in the Examples presented herein, biomarker panels of the invention were able to distinguish between patients with colorectal cancer and patients with various non-cancerous colon and bowel diseases and healthy patients (see **Figures 2** and **3**). For example, using a three-assay panel comprising measurements of nucleosomes *per se,* nucleosomes containing histone isoform H3.1 and IL-6, with a model and cut-offs optimized to discriminate between patients with CRC both from subjects with no findings on colonoscopy and from patients with non-malignant benign colon or bowel disease, the method of the invention was able to identify 50% of CRC cases at 90% specificity among all other patients (diseased and non-diseased) with a clear disease stage dependence as shown in **Figure 3****.** This embodiment of the invention is therefore useful for the testing of persons with symptoms of colorectal disease to identify patients with CRC from those with other non-malignant conditions or no disease.

As another example, differential diagnosis may be conducted on patients with suspected ovarian cancer compared to a non-cancerous disease. Women may have a pelvic mass of unknown aetiology. Such a mass may be malignant but may also be a cyst or fibroid in nature due to a variety of other causes. Non-cancerous diseases of the ovaries include, but are not limited to: endometriosis, ovarian cysts and polycystic ovary syndrome. The invention may be used in diagnosing patients with suspected ovarian cancer (*e.g*. due to symptoms) because the test may differentiate between patients with ovarian cancer and other non-cancerous diseases. Therefore, in one embodiment, the non-cancerous disease is a pelvic mass or a non-cancerous disease of the ovaries with similar signs and/or symptoms to ovarian cancer.

As another example, differential diagnosis may be conducted on patients with suspected prostate cancer compared to a non-cancerous prostate disease. Non-cancerous diseases of the prostate include, but are not limited to: prostate enlargement or prostatitis. The invention may be used in diagnosing patients with suspected prostate cancer (e.g. due to symptoms) because the test may differentiate between patients with prostate cancer and other non-cancerous diseases. Therefore, in one embodiment, the non-cancerous prostate disease is a disease which has similar signs and/or symptoms to prostate cancer.

In one embodiment, the control comprises a healthy subject, a non-diseased subject and/or a subject without cancer. In one embodiment, the method comprises comparing the amount of biomarker(s) present in a body fluid sample obtained from the subject with the amount of biomarker(s) present in a body fluid sample obtained from a normal subject. It will be understood that a "normal" subject refers to a healthy/non-diseased subject.

In one embodiment, the control comprises a subject with a non-cancerous disease. Methods of the invention are able to distinguish between subjects with cancer and subjects with non-cancerous diseases, such as COPD (when compared to lung cancer) and colitis or diverticulosis (when compared to CRC), as described in the methods of differential diagnosis section provided herein. Therefore, in one aspect the diagnosis comprises differential diagnosis of cancer from a non-cancerous disease.

### Methods of patient assessment

The invention finds particular use in assessing whether a patient requires further investigation for the cancer (*e.g*. for diagnosis and/or identification of organ location). Such procedures, including colonoscopies, other endoscopy methods, mammographies, X-rays, LDCT scans, other scans and biopsies are invasive or potentially hazardous and are relatively costly to healthcare providers. Therefore there is a need to reduce the number of patients sent for unnecessary investigations. For example, this aspect of the invention will be useful for assessing FIT or LDCT positive persons as in need of a colonoscopy or a biopsy. Therefore, according to a further aspect of the invention, there is provided a method for assessing the suitability of a patient for cancer investigations (*i.e*. determining whether a patient requires further cancer investigative tests), comprising:
detecting or measuring IL-6 and histone isoform H3.1, in a body fluid sample obtained from the patient; and
using the level or concentration of IL-6 and histone isoform H3.1 detected to determine whether the patient requires further cancer investigations.

As shown in the Examples provided herein, the invention has application as a CRC blood test for use to detect CRC in asymptomatic subjects, in patients that are non-compliant with FIT, or in addition to FIT for lower combined false positive rates. A positive result in the test of the invention would, like FIT, indicate the need for referral for a colonoscopy.

According to a further aspect of the invention there is provided a method of identifying a patient in need of a colonoscopy comprising applying a body fluid sample obtained from the patient to a panel test as defined herein, and using the results obtained from the panel test to identify whether the patient is in need of a colonoscopy.

According to a further aspect of the invention there is provided a method of identifying a patient in need of a LDCT, mammography or other scan comprising applying a body fluid sample obtained from the patient to a panel test as defined herein, and using the results obtained from the panel test to identify whether the patient is in need of a scan.

In one embodiment, the method described herein is repeated on multiple occasions. This embodiment provides the advantage of allowing the detection results to be monitored over a time period. Such an arrangement will provide the benefit of monitoring or assessing the efficacy of treatment of a disease state. Such monitoring methods of the invention can be used to monitor onset, progression, stabilisation, amelioration, relapse and/or remission.

Thus, the invention also provides a method of monitoring efficacy of a therapy for a disease state in a subject, suspected of having such a disease, comprising detecting and/or quantifying the biomarker (e.g. biomarker panel described herein) present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the nature or amount of the biomarker(s) in test samples taken on different occasions.

Thus, according to a further aspect of the invention, there is provided a method for monitoring efficacy of therapy for a disease state in a human or animal subject, comprising:
(a) quantifying the panel biomarkers as defined herein; and
(b) comparing the panel result in a test sample with that of one or more control(s) and/or one or more previous test sample(s) taken at an earlier time from the same test subject.

A change in the biomarker result in the test sample relative to the level in a previous test sample taken earlier from the same test subject may be indicative of a beneficial effect, *e.g*. stabilisation or improvement, of said therapy on the disorder or suspected disorder. Furthermore, once treatment has been completed, the method of the invention may be periodically repeated in order to monitor for the recurrence of a disease.

Methods for monitoring efficacy of a therapy can be used to monitor the therapeutic effectiveness of existing therapies and new therapies in human subjects and in non-human animals (*e.g.* in animal models). These monitoring methods can be incorporated into screens for new drug substances and combinations of substances.

In a further embodiment, the monitoring of more rapid changes due to fast acting therapies may be conducted at shorter intervals of hours or days.

### Panel tests

The combination of markers described herein may be used to prepare a panel test, in particular for use in the diagnosis of cancer and/or monitoring of patients with cancer or suspected cancer.

Therefore, according to a further aspect of the invention, there is provided a kit comprising reagents to detect IL-6 and histone isoform H3.1. The kits described herein may be for use in the diagnosis of cancer, such as lung, colorectal, ovarian and/or prostate cancer.

According to a further aspect of the invention, there is provided a kit comprising reagents to detect IL-6, histone H3.1 and optionally one or more biomarkers selected from the list consisting of: IL-8, IL-10, nucleosomes or a component thereof, and an epigenetic feature of a nucleosome.

In a further embodiment, the kit comprises (optionally among others) reagents to detect IL-6, histone H3.1 and the (total) level of cell free nucleosomes in a sample.

In one embodiment, the kit additionally comprises reagents to detect one or more biomarkers selected from the group consisting of: IL-8, IL-10, nucleosomes or a component thereof, and an epigenetic feature of a nucleosome. In one embodiment, the kit is for use with a body fluid sample obtained from the patient.

As shown in the Examples provided herein, a panel assay comprising measurements of nucleosomes containing histone isoform H3.1 and IL-6 was able to detect 77% of lung cancer cases from normal donors as shown in **Table 2** and also from those with COPD as shown in **Figure 1****.** These panel assays thus have high sensitivity and specificity and application for use as a lung cancer test for use in high risk groups, for example long term heavy smokers, or for patients that are non-compliant with low dose computed tomography (LDCT), or in place of repeated LDCT scans for monitoring of patients with nodules of unknown aetiology to avoid repeated dangerous x-ray exposure or as an adjunct test to LDCT to help in the investigation of false positive results generated by LDCT screening due to low specificity. Use of a panel comprising measurements of nucleosomes *per se,* nucleosomes containing histone isoform H3.1 and IL-6 was able to identify 80% of CRC cases at 89% specificity as shown in **Figure 2**. This accuracy is comparable to the accuracy of the FIT CRC screening test, with sensitivity of around 72% at a specificity of 95%, and has application for the detection of CRC.

In one embodiment, the kit additionally comprises reagents to measure the levels of fecal haemoglobin. As described in the Examples, the invention may be used in combination with the fecal haemoglobin level to increase the specificity of the FIT test.

According to a further aspect of the invention there is provided the *in vitro* use of the kit as defined herein to identify a patient in need of treatment for cancer.

According to a further aspect of the invention there is provided the *in vitro* use of the kit as defined herein to monitor a patient for progression of cancer (*e.g*. further growth of the tumour, or advancement to a different stage of cancer). Embodiments of this aspect include *in vitro* use to detect disease progression in watchful waiting, active surveillance and monitoring postsurgery or other treatment for relapse.

According to a further aspect of the invention there is provided the *in vitro* use of the kit as defined herein to evaluate the effectiveness of a cancer treatment in a patient.

According to a further aspect of the invention there is provided the *in vitro* use of the kit as defined herein to select a treatment for a patient with cancer.

### Measurement methods

In one embodiment, the level or concentration of IL-6 and histone isoform H3.1 detected is compared to a control. It will be clear to those skilled in the art that the control subjects may be selected on a variety of basis which may include, for example, subjects known to be free of the disease or may be subjects with a different disease (for example, for the investigation of differential diagnosis). The "control" may comprise a healthy subject, a non-diseased subject and/or a subject without cancer. The control may also be a subject with a different stage of cancer, **e.g.** stage I, stage II, stage III or stage IV cancer. Comparison with a control is well known in the field of diagnostics.

It will be understood that it is not necessary to measure healthy/non-diseased controls for comparative purposes on every occasion because once the 'normal range' is established it can be used as a benchmark for all subsequent tests. A normal range can be established by obtaining samples from multiple control subjects without cancer and testing for the level of biomarker. Results (*i.e.* biomarker levels) for subjects suspected to have cancer can then be examined to see if they fall within, or outside of, the respective normal range. Use of a 'normal range' is standard practice for the detection of disease.

If a subject is determined to not have cancer, then the invention may still be used for the purposes of monitoring disease progression. For example, if the use comprises a blood, serum or plasma sample from a subject determined not to have cancer, then the biomarker level measurements can be repeated at another time point to establish if the biomarker level has changed.

References to "subject" or "patient" are used interchangeably herein. In one embodiment, the patient is a human patient. In one embodiment, the patient is a (non-human) animal.

In one embodiment, detection or measurement of IL-6 and histone isoform H3.1 comprises an immunoassay, immunochemical, mass spectroscopy, chromatographic, chromatin immunoprecipitation or biosensor method.

In one embodiment, the detection or measurement comprises an immunoassay. In a preferred embodiment of the invention there is provided a 2-site immunoassay method for cytokine and/or nucleosome moieties. In particular, such a method is preferred for the measurement of nucleosomes or nucleosome incorporated epigenetic features *in situ* employing two anti-nucleosome binding agents or an anti-nucleosome binding agent in combination with an anti-histone modification or anti-histone variant or anti-DNA modification or anti-adducted protein detection binding agent. In another embodiment of the invention, there is provided a 2-site immunoassay employing a labelled anti-nucleosome detection binding agent in combination with an immobilized anti-histone modification or anti-histone variant or anti-DNA modification or anti-adducted protein binding agent.

Detecting or measuring the level of the biomarker(s) may be performed using one or more reagents, such as a suitable binding agent. In one embodiment, the one or more binding agents comprises a ligand or binder specific for the desired biomarker, e.g. IL-8, IL-6, IL-10, nucleosomes or component part thereof, an epigenetic feature of a nucleosome, or a structural/shape mimic of the nucleosome or component part thereof. The term "biomarker" as defined herein includes any single biomarker moiety or a combination of individual biomarker moieties in a biomarker panel.

It will be clear to those skilled in the art that the terms "antibody", "binder" or "ligand" in regard to any aspect of the invention is not limiting but intended to include any binder capable of binding to particular molecules or entities and that any suitable binder can be used in the method of the invention. It will also be clear that the term "nucleosomes" is intended to include mononucleosomes and oligonucleosomes and any protein-DNA chromatin fragments that can be analysed in fluid media.

Methods of detecting biomarkers are known in the art. In one embodiment, the reagents comprise one or more ligands or binders. In one embodiment, the ligands or binders include naturally occurring or chemically synthesised compounds, capable of specific binding to the desired target. A ligand or binder may comprise a peptide, an antibody or a fragment thereof, or a synthetic ligand such as a plastic antibody, or an aptamer or oligonucleotide, capable of specific binding to the desired target. The antibody can be a monoclonal antibody or a fragment thereof. It will be understood that if an antibody fragment is used then it retains the ability to bind the biomarker so that the biomarker may be detected (in accordance with the present invention). A ligand/binder may be labelled with a detectable marker, such as a luminescent, fluorescent, enzyme or radioactive marker; alternatively or additionally a ligand according to the invention may be labelled with an affinity tag, *e.g.* a biotin, avidin, streptavidin or His (*e.g.* hexa-His) tag. Alternatively, ligand binding may be determined using a label-free technology for example that of ForteBio Inc.

Diagnostic or monitoring kits (or panels) are provided for performing methods of the invention. Such kits will suitably comprise one or more ligands for detection and/or quantification of the biomarkers, and/or a biosensor, and/or an array as described herein, optionally together with instructions for use of the kit.

A further aspect of the invention is a kit for detecting the presence of a disease state, comprising a biosensor capable of detecting and/or quantifying one or more of the biomarkers as defined herein. As used herein, the term "biosensor" means anything capable of detecting the presence of the biomarker. Examples of biosensors are described herein. Biosensors may comprise a ligand binder or ligands, as described herein, capable of specific binding to the biomarker. Such biosensors are useful in detecting and/or quantifying the biomarkers.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, *e.g*. in the ward, outpatients' department, surgery, home, field and workplace. Biosensors to detect one or more biomarkers of the invention include acoustic, plasmon resonance, holographic, Bio-Layer Interferometry (BLI) and microengineered sensors. Imprinted recognition elements, thin film transistor technology, magnetic acoustic resonator devices and other novel acousto-electrical systems may be employed in biosensors for detection of the one or more biomarkers.

Biomarkers for detecting the presence of a disease are essential targets for discovery of novel targets and drug molecules that retard or halt progression of the disorder. As the result for a biomarker or biomarker panel is indicative of disorder and of drug response, the biomarker is useful for identification of novel therapeutic compounds in *in vitro* and/or *in vivo* assays. Biomarkers and biomarker panels can be employed in methods for screening for compounds that modulate the activity of the biomarker.

Thus, in a further aspect of the invention, there is provided the *in vitro* use of a binder or ligand, as described, which can be a peptide, antibody or fragment thereof or aptamer or oligonucleotide directed to the biomarkers; or the *in vitro* use of a biosensor, or an array, or a kit according to the invention, to identify a substance capable of promoting and/or of suppressing the generation of the biomarker.

The term "biomarker" means a distinctive biological or biologically derived indicator of a process, event, or condition. Biomarkers can be used in methods of diagnosis, *e.g.* clinical screening, and prognosis assessment and in monitoring the results of therapy, identifying subjects most likely to respond to a particular therapeutic treatment, drug screening and development. Biomarkers and uses thereof are valuable for identification of new drug treatments and for discovery of new targets for drug treatment.

The term "detecting" or "diagnosing" as used herein encompasses identification, confirmation, and/or characterisation of a disease state. Methods of detecting, monitoring and of diagnosis according to the invention are useful to confirm the existence of a disease, to monitor development of the disease by assessing onset and progression, or to assess amelioration or regression of the disease. Methods of detecting, monitoring and of diagnosis are also useful in methods for assessment of clinical screening, prognosis, choice of therapy, evaluation of therapeutic benefit, *i.e.* for drug screening and drug development.

Identifying and/or quantifying can be performed by any method suitable to identify the presence and/or amount of a specific protein in a biological sample from a subject or a purification or extract of a biological sample or a dilution thereof. In methods of the invention, quantifying may be performed by measuring the concentration of the target in the sample or samples. Biological samples that may be tested in a method of the invention include those as defined hereinbefore. The samples can be prepared, for example where appropriate diluted or concentrated, and stored in the usual manner.

Identification and/or quantification of biomarkers may be performed by detection of the biomarker or of a fragment thereof, *e.g.* a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length. It is noted in particular that peptides of the same or related sequence to that of histone tails are particularly useful fragments of histone proteins.

For example, detecting and/or quantifying can be performed using an immunological method, such as an immunoassay. Immunoassays include any method employing one or more antibodies or other specific binders directed to bind to the biomarkers defined herein. Immunoassays include 2-site immunoassays or immunometric assays employing enzyme detection methods (for example ELISA), fluorescence labelled immunometric assays, time-resolved fluorescence labelled immunometric assays, chemiluminescent immunometric assays, immunoturbidimetric assays, particulate labelled immunometric assays and immunoradiometric assays as well as single-site immunoassays, reagent limited immunoassays, competitive immunoassay methods including labelled antigen and labelled antibody single antibody immunoassay methods with a variety of label types including radioactive, enzyme, fluorescent, time-resolved fluorescent and particulate labels. All of said immunoassay methods are well known in the art, including for cytokines and for nucleosomes.

In another example, detecting and/or quantifying can be performed by one or more method(s) selected from the group consisting of: SELDI (-TOF), MALDI (-TOF), a 1-D gel-based analysis, a 2-D gel-based analysis, Mass spectrometry (MS), reverse phase (RP) LC, size permeation (gel filtration), ion exchange, affinity, HPLC, UPLC and other LC or LC MS-based techniques. Appropriate LC MS techniques include ICAT^{®} (Applied Biosystems, CA, USA), or iTRAQ^{®} (Applied Biosystems, CA, USA). Liquid chromatography (*e.g*. high pressure liquid chromatography (HPLC) or low pressure liquid chromatography (LPLC)), thin-layer chromatography, NMR (nuclear magnetic resonance) spectroscopy could also be used.

Methods involving identification and/or quantification of one or more biomarkers of the invention can be performed on bench-top instruments, or can be incorporated onto disposable, diagnostic or monitoring platforms that can be used in a non-laboratory environment, *e.g*. in the physician's office or at the subject's bedside. Suitable biosensors for performing methods of the invention include "credit" cards with optical or acoustic readers. Biosensors can be configured to allow the data collected to be electronically transmitted to the physician for interpretation and thus can form the basis for e-medicine.

The identification of biomarkers for a disease state permits integration of diagnostic procedures and therapeutic regimes. Detection of a biomarker can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized therapies, as assessment of response can be used to fine-tune dosage, minimise the number of prescribed medications, reduce the delay in attaining effective therapy and avoid adverse drug reactions. Thus by monitoring a biomarker, subject care can be tailored precisely to match the needs determined by the disorder and the pharmacogenomic profile of the subject, the biomarker can thus be used to titrate the optimal dose, predict a positive therapeutic response and identify those subjects at high risk of severe side effects.

Biomarker-based tests provide a first line assessment of 'new' subjects, and provide objective measures for accurate and rapid diagnosis, not achievable using the current measures.

Biomarker monitoring methods, biosensors and kits are also vital as subject monitoring tools, to enable the physician to determine whether relapse is due to worsening of the disorder. If pharmacological treatment is assessed to be inadequate, then therapy can be reinstated or increased; a change in therapy can be given if appropriate. As the biomarkers are sensitive to the state of the disorder, they provide an indication of the impact of drug therapy.

It will be understood that the embodiments described herein may be applied to all aspects of the invention, *i.e.* the embodiment described for the uses may equally apply to the claimed methods and so forth.

The invention will now be illustrated with reference to the following non-limiting examples.

### EXAMPLES

### Example 1

Blood (plasma) samples were taken from 144 people including 47 patients with lung cancer (small cell lung cancer and non-small cell lung cancer), 43 normal donors of similar age and 54 patients with chronic obstructive pulmonary disease (COPD). Measurements of nucleosomes containing histone isoform H3.1 and IL-10 were made by ELISA. In brief, measurements of nucleosomes containing histone isoform H3.1 were made as follows: 80µl assay buffer and 20µl of plasma sample or standard nucleosome preparation was added to a microtiter well coated with an antibody directed to bind to histone H3.1. The microtiter plate was covered and incubated with gentle shaking for 2.5 hours at room temperature. The contents of the microtiter wells were discarded. The wells were washed three times with 200µl of a wash solution and 100µl of a biotinylated anti-nucleosome antibody was added. The microtiter plate was again covered and incubated with gentle shaking for 1.5 hours at room temperature. The contents of the microtiter wells were discarded. The wells were washed three times with 200µl of a wash solution and 100µl of a streptavidin-HRP solution was added. The microtiter plate was again covered and incubated with gentle shaking for 0.5 hours at room temperature. The contents of the microtiter wells were discarded. The wells were washed three times with 200µl of a wash solution and 100µl of a HRP (horse radish peroxidase) substrate solution was added. The microtiter plate was covered and incubated with gentle shaking for 20 minutes in the dark at room temperature. The absorbance (OD) of the wells was measured at 405nm. The OD levels were either used directly or the plasma level of nucleosomes containing histone H3.1 were interpolated from a standard curve. Plasma IL-10 levels were measured using a commercially available ELISA method.

We modelled the assay results by Logistic Regression analysis to train for the model or algorithm with the highest AUC for a comparison of patients with lung cancer vs normal donors. The results showed that IL-10 results could be combined with results for nucleosomes containing histone H3.1 as an effective assay panel with an associated algorithm for cancer detection. The algorithm was able to discriminate 68% of lung cancer patients from normal donors at 93% specificity, as shown in **Table 2.**

### Example 2

Plasma samples taken from the same 144 people described in Example 1 were assayed for nucleosomes containing histone isoform H3.1 as described in Example 1 and for IL-6 and IL-8 using a commercially available ELISA method. We modelled the assay results by Logistic Regression analysis to train for the model or algorithm with the highest AUC for a comparison of patients with lung cancer vs normal donors. The results showed that IL-6 results could be combined with results for nucleosomes containing histone H3.1 as an effective assay panel with an associated algorithm for lung cancer detection. The algorithm was able to detect 77% of lung cancer cases from normal donors at 90% specificity as shown in **Table 2** and also from those with COPD as shown in **Figure 1****.**

**Table 2. Individual biomarker and panel biomarker results for accuracy of lung cancer detection (lung cancer vs normal donors)**

| | AUC (%) | Sensitivity (%) at 90% specificity | Sensitivity (%) at 80% specificity |
|---|---|---|---|
| H3.1-nucleosomes (H3.1-nucs) | 79 | 45 | 66 |
| IL-6 | 79 | 53 | 64 |
| IL-8 | 66 | 40 | 55 |
| IL-10 | 78 | 45 | 68 |
| Panel: IL-10, H3.1-nucs | 88 | 68 | 77 |
| Panel trained on lung cancer only: IL-6, H3.1-nucs | 86 | 77 | 77 |
| Panel trained lung/CRC combined model: IL-6, H3.1-nucs | 86 | 77 | 83 |

### Example 3

Blood (plasma) samples were taken from 100 people including 20 patients with colorectal cancer (CRC), 62 patients with a variety of non-malignant diseases of the colon including colitis, Crohn's disease and diverticulosis and 18 patients with gastroenterological symptoms but no findings on colonoscopy. We made measurements of nucleosomes containing histone isoform H3.1 as described in Example 1 and for IL-6, IL-8 and IL-10 using a commercially available ELISA method. We also measured nucleosomes *per se* using a similar method to that described above for nucleosomes containing histone H3.1, but using an anti-H3 antibody coated onto the microtiter wells. We modelled the assay results by Logistic Regression analysis to train for the model or algorithm with the highest AUC for a comparison of patients with CRC vs patients with no findings on colonoscopy. The results showed that IL-6 results could be combined with results for nucleosomes *per se* and nucleosomes containing histone H3.1 as an effective assay panel with an associated algorithm for cancer detection. The algorithm was able to detect 80% of CRC cancer cases from patients with no findings at 89% specificity with a clear disease stage dependence as shown in **Table 3** and **Figure 2****.**

**Table 3. Individual biomarker and panel biomarker results for accuracy of CRC detection (CRC vs symptomatic subjects with no findings on colonoscopy)**

| | AUC (%) | Sensitivity (%) at 90% specificity | Sensitivity (%) at 80% specificity |
|---|---|---|---|
| Nucleosomes *per se* (nucs *per se)* | 71 | 40 | 45 |
| H3.1-nucleosomes (H3.1-nucs) | 72 | 45 | 65 |
| IL-6 | 83 | 40 | 75 |
| IL-8 | 66 | 20 | 45 |
| IL-10 | 74 | 15 | 45 |
| Panel trained cancer v no findings: IL-6, nucs *per se,* H3.1-nucs | 84 | 80 (89% specificity) | 80 |
| Panel trained cancer v no findings + benign: IL-6, nucs *per se,* H3.1-nucs | 74 | 50 | 60 |
| Panel: IL-6, nucs *per se* | 85 | 45 | 80 |
| Panel trained CRC only: IL-6, H3.1-nucs | 84 | 45 | 80 |
| Panel trained lung/CRC combined model: IL-6, H3.1-nucs | 84 | 45 | 80 |

### Example 4

We used a three-assay panel comprising the same three measurements of nucleosomes *per se*, nucleosomes containing histone isoform H3.1 and IL-6 as described in Example 3 on the same 100 patients described in Example 3, but we modelled the assay results by Logistic Regression analysis to train for the model or algorithm with the highest AUC for a comparison of patients with CRC vs both patients with no findings on colonoscopy and patients with a non-malignant benign colon or bowel disease. The method of the invention was able to identify 50% of CRC cases at 90% specificity among all other patients (diseased and non-diseased) with a clear disease stage dependence as shown in **Figure 3****.** The embodiment of the invention described in here in Example 4 is useful for the testing of persons with symptoms of colorectal disease to identify patients with CRC from those with other non-malignant conditions or no disease.

The results for Examples 3 and 4 show that the accuracy in terms of sensitivity and specificity of methods of the invention can be tailored to maximise sensitivity for a test to be used in asymptomatic subjects, or to maximise specificity for a test to be used in symptomatic patients to avoid false positive cancer diagnoses among patients with benign diseases by alternative model training techniques. This facility allows tailoring of the method for use with symptomatic or non-symptomatic patients or other applications.

### Example 5

We hypothesized that panel blood tests including levels of both cytokine interleukin molecules and nucleosome moieties are useful, not only as markers of lung or colorectal cancers individually, but of cancers generally. To test this hypothesis, we measured nucleosomes containing histone isoform H3.1 and IL-6 by ELISA as described above in the 244 people included in the combined 144 person cohort for lung cancer and the 100 person cohort for CRC described in Examples 1 and 3. We then used the combined data from the two cohorts to model the assay results by Logistic Regression analysis to train for the model or algorithm with the highest AUC for a comparison of patients with lung cancer or CRC vs patients with no findings on colonoscopy and normal donors. This training produced an associated regression model that gave a combined accuracy for the detection of either lung or colorectal cancer of 75% sensitivity at a specificity of 90% or 81% sensitivity at a specificity of 80%.

We also tested the efficacy of this method of the invention for the detection of CRC and lung cancer separately in each of the two individual CRC and lung cancer (training) cohorts. In the lung cancer cohort, the individual result was a 77% sensitivity for detection of lung cancer at a specificity of 90% or 83% sensitivity at a specificity of 80% (see **Table 2**). In the CRC cohort the individual result was a 45% sensitivity for detection of CRC at a specificity of 90% or 80% sensitivity at a specificity of 80% (see **Table 3**). Thus, the method of the invention trained on CRC and lung cancer combined is equally efficient for the detection of either disease separately as the methods trained on the respective individual diseases.

### Example 6

In order to further test the hypothesis that a panel blood test including levels of both cytokine interleukin molecules and nucleosome moieties is useful as a blood test for the detection of cancer generally, we applied the combined model developed in the two training cohorts, to two further validation patient cohorts collected independently of the training cohorts in a different country. First, we measured nucleosomes containing histone isoform H3.1 and IL-6 by ELISA in another independently collected lung cancer validation cohort of 70 people including 30 lung cancer patients (both small cell and non-small cell lung cancer), 30 normal donor subjects and 10 patients with COPD. We then calculated the algorithm score for these 70 people, using the algorithm developed on the combined 244 person lung cancer and CRC training cohort in Example 5. The algorithm scores for the 70 people showed that the 2-assay combined panel was able to detect 93% of lung cancers at a specificity of 93% among normal donors and 100% of lung cancers at a specificity of 80% in the validation cohort as shown in **Figure 4****.** Surprisingly, the observed accuracy of the method of the invention is higher in the validation cohort than that observed in the training set. This may be related to cancer stage and the mix of patients with small cell and non-small disease in the two cohorts. The results demonstrate the validity and utility of the method of the invention.

### Example 7

We measured nucleosomes containing histone isoform H3.1 and IL-6 by ELISA in an independently collected multiple cancer validation cohort of 63 people including 30 patients with a variety of cancers including cancer of the lung, colon, rectum, stomach, kidney, prostate, breast, pharynx, larynx, ovary, oesophagus and bladder as well as 33 normal donor subjects. This was done to determine whether the 2-assay panel combined model could detect a variety of different cancer diseases beyond CRC and lung cancer. We then calculated the algorithm score for these 63 people, using the algorithm developed on the combined 244 person lung cancer and CRC training cohort in Example 5. The algorithm scores for the 63 people showed that the 2-assay combined panel was able to detect 47% of cancers overall at 90% specificity including cancer of the kidney (1 of 2 cases or 1/2), larynx (2/3), lung (1/5), oesophagus (1/1), ovary (6/8), prostate (2/4) and rectum (1/2). At 80% specificity, the 2-assay combined panel was able to detect 67% of cancers overall and every type of cancer tested except stomach, including cancer of the bladder (1/1), breast (1/1), kidney (1/2), larynx (2/3), lung (2/5), oesophagus (1/1), ovary (7/8), pharynx (1/2), prostate (3/4), rectum (1/2) and stomach (0/1). There was only a single stomach cancer patient included in the cohort, therefore inclusion of further stomach cancer patients is expected to lead to increased detection rates. The results are shown in **Figure 5** and demonstrate the breadth of utility of the methods of the invention to detect multiple cancer types with utility as a test for cancer *per se.*

### Example 8

We measured nucleosomes *per se* and IL-6 in the 100 person CRC cohort described in Example 3 including 20 patients with CRC, 62 patients with a variety of non-malignant diseases of the colon including colitis, Crohn's disease and diverticulosis and 18 patients with gastroenterological symptoms but no findings on colonoscopy. We modelled the assay results by Logistic Regression analysis to train for the model or algorithm with the highest AUC for a comparison of patients with CRC vs patients with no findings on colonoscopy. The method of the invention was able to identify 45% of CRC cases at 90% specificity among patients with no findings on colonoscopy. This embodiment of the invention is useful for the testing of persons with CRC from those with no disease.

In a similar way to the experiment described in Example 7, this model was then applied to the validation cohort including patients with multiple cancer diseases. This 2-assay panel including IL-6 and nucleosomes *per* se was able to detect 60% of cancers at 90% specificity including cancer of the kidney (1/2), larynx (2/3), lung (1/5), oesophagus (1/1), ovary (8/8), pharynx (1/2), prostate (3/4), rectum (1/2). At 80% specificity, this 2-assay panel detected 73% of all the various cancer cases and every type of cancer tested except stomach, including cancer of the bladder (1/1), breast (1/1), kidney (1/2), larynx (2/3), lung (2/5), oesophagus (1/1), ovary (8/8), pharynx (1/2), prostate (4/4), rectum (1/2) and stomach (0/1). The results are shown in **Figure 6** and demonstrate the breadth of utility of the methods of the invention to detect multiple cancer types with utility as a test for cancer *per se.*

### Example 9

To further demonstrate the method of the invention we trained another three-assay panel model including measurements of nucleosomes *per se*, nucleosomes containing histone isoform H3.1 and IL-6 on the results for the CRC training cohort described in Examples 3 and 8. This model has an accuracy of 80% sensitivity at 89% specificity or 80% sensitivity at 80% specificity for CRC detection (**Table 3**). In a similar way to the experiment described in Example 7, this model was then applied to the validation cohort including patients with multiple cancer diseases. This 3-assay panel was able to detect 37% of cancers at 90% specificity including cancer of the kidney (1/2), lung (2/5), ovary (5/8), prostate (1/4), rectum (1/2) and stomach (1/1). At 80% specificity, the 3-assay panel detected 57% of all the various cancer cases and every type of cancer tested except breast, including cancer of the bladder (1/1), kidney (2/2), larynx (1/3), lung (3/5), oesophagus (1/1), ovary (6/8), pharynx (2/2), prostate (2/4), rectum (1/2) and stomach (1/1). The results are shown in **Figure 7** and demonstrate the breadth of utility of the methods of the invention to detect multiple cancer types with utility as a test for cancer *per se.*

### Example 10

To demonstrate the method of the invention using simple cut-off values, rather than models or algorithms based on regression analysis of results, we reanalysed the joint data from the 144 people including 47 patients with lung cancer described in Example 1, plus the 100 people including 20 patients with colorectal cancer (CRC) described in Example 3 plus the 70 people including 30 lung cancer patients described in Example 6 (314 subjects in total including 97 patients with cancer) using simple positive negative cut-off values for a 2-assay panel including IL-6 and nucleosomes containing histone isoform H3.1. The cut-off for IL-6 was set at ≥4 pg/ml and the cut-off for nucleosomes containing histone isoform H3.1 was set at an assay response of Optical Density of ≥1.2 OD units (equivalent to approximately ≥200 ng/ml). Any sample which found to have at least one positive result (above the respective threshold cut-off value) was deemed positive. This analysis detected 71% of the cancer cases at a specificity of 93%. This is very close to the accuracy of the FIT test which is widely used as a front-line screening test for (colorectal) cancer.

The advantages of using simple cut-off values in a panel test include the ease with which clinicians are able to understand the test and the elimination of any need for software or other aids in the interpretation of the test results.

### Example 11

Plasma samples were collected from 135 patients referred for colonoscopy after testing positive for FIT using the OC-Sensor FIT test (fecal haemoglobin level ≥100ng/ml). The patients included asymptomatic screening patients, symptomatic patients and patients under surveillance for CRC relapse or disease progression. Of the 135 patients, 41 patients were found to have no lesion on colonoscopy, 37 patients had one or more non-advanced adenomas, 35 patients had one or more advanced adenomas and 22 patients had CRC of whom 5 were diagnosed with stage I disease, 2 with stage II, 8 with stage III, 6 with stage IV and 1 with unknown stage disease.

We tested the plasma samples for levels of nucleosomes containing histone isoform H3.1, IL-6, IL-8, IL-10, CRP and HMGB1. The numerical FIT levels (ng/ml) and blood plasma results were analysed using ROC analysis of CRC versus persons with no findings on colonoscopy, to determine the specificity of the assay or model at which the sensitivity reaches 100% (i.e. the proportion of people in whom no CRC or adenoma was found on colonoscopy who were correctly identified as negative, whilst correctly identifying all 22 patients with CRC as positive). This provides a figure for the potential reduction in colonoscopies that may be achieved whilst prioritising all the patients with cancer for colonoscopies by a triage blood test of the invention. All of the individual assays performed had positive AUCs and were able to correctly identify some patients with no colorectal lesion with a zero false negative rate for CRC. Table 4 shows the specificity of the biomarker for CRC at 100% sensitivity. This is a measure of the proportion (%) of patients with no findings on colonoscopy correctly predicted not to have CRC whilst missing zero cancer cases.

**Table 4. Performance of individual assays for triage of FIT positive subjects**

| Assay | AUC (%) | Specificity (%) at 100% sensitivity |
|---|---|---|
| FIT | 86 | 32 |
| H3.1-nucleosomes | 68 | 5 |
| IL-6 | 59 | 5 |
| 1L-8 | 66 | 3 |
| IL-10 | 53 | 3 |
| CRP | 71 | 12 |
| HMGB1 | 53 | 8 |

The blood assay results were then combined with FIT results to produce algorithms by regression analysis for the identification of persons who do not have CRC whilst maintaining a zero false negative rate for CRC. Some of the resulting models or algorithms developed are shown in Table 5.

**Table 5. Performance of FIT/blood combination assay models for triage of FIT positive subjects**

| Model | AUC (%) | Specificity (%) at 100% sensitivity | |
|---|---|---|---|
| | | All subjects | No surveillance subjects |
| FIT + H3.1-nucleosomes | 84 | 18 | 49 |
| FIT + IL-6 | 86 | 44 | 68 |
| FIT + IL-8 | 85 | 53 | 55 |
| FIT + H3.1-nucleosomes + IL-6 | 85 | 17 | 56 |
| FIT + IL-6 + IL-8 | 87 | 55 | 61 |
| FIT + H3.1-nucleosomes + IL-6 + IL-8 | 88 | 58 | 61 |

Use of the numerical value of the FIT test alone identified 32% of patients with blood in their stool but in whom no lesion was found on colonoscopy. However, use of methods of the invention were able to increase this to up to 58%. When the derived models were applied to the symptomatic and asymptomatic subjects tested (without the surveillance subjects), the proportion of people with no colorectal lesion correctly identified as negative rose further to up to 68%.

### REFERENCES

Allin et al, Crit Rev Clin Lab Sci, 48(4): 155-170, 2011
Bjorkman et al, Scandinavian J Immunol, 57: 525-533, 2003
Chadha et al, Clin Cancer Investig J, 3: 72-79, 2014
Du et al, Cancer Chemother Pharmacol, 81: 1111-1119, 2018
Herranz and Esteller, Methods Mol Biol, 361: 25-62, 2007
Holdenrieder et al, Int J Cancer, 95: 114-20, 2001
Holdenrieder et al, Clin Chem, 51(6): 1026-1029, 2005
Holdenrieder and Stieber, Crit Rev Clin Lab Sci, 46(1): 1-24, 2009
Lipitz and Harris, Oncoimmunol, 5: e1093722, 2016
Madej-Michniewicz et al, Nat Sci Rep, 5: 14382, 2015
Midthun, F1000Res, 5: F1000 Faculty Rev-739, 2016
Moyer, Ann Intern Med, 160(5): 330-338, 2014
Potter et al, Clin Chem, 60(9): 1183-1191, 2014
Salgame et al, Nucleic Acids Res, 25(3): 680-681, 1997
Snyder et al, Cell, 164: 57-68, 2016
Taniguchi and Karin, Semin Immunol, 26: 54-74, 2014
van Nieuwenhuijze et al, Ann Rheum Dis, 62: 10-14, 2003
Wang and Sun, Int J Onocology, 44: 1032-1040, 2014
Wojtacki et al, Neoplasma, 41(4): 213-6, 1994
Xia et al, PLoS ONE, 10: e0123484, 2015
Xie, Cytokine Growth Factor Rev, 12: 375-391, 2001
Yang et al, Nucleic Acids Res, 32: e38, 2004
Zhao and Garcia, Cold Spring Harb Perspect Biol, 7: a025064, 2015

## Claims

1. *In vitro* use of a panel of biomarkers in a body fluid sample for diagnosing and/or monitoring a cancer, wherein the biomarkers comprise histone isoform H3.1 and Interleukin-6 (IL-6).

2. The *in vitro* use as defined in claim 1, wherein the panel of biomarkers additionally comprise Interleukin-8 (IL-8) and Interleukin-10 (IL-10).

3. The *in vitro* use as defined in claim 1 or claim 2, wherein the body fluid sample is a blood, serum or plasma sample.

4. The *in vitro* use as defined in any one of claims 1 to 3, wherein histone isoform H3.1 and IL-6 are measured by an immunoassay or mass spectroscopy.

5. The *in vitro* use as defined in any one of claims 1 to 4, wherein the cancer is selected from: lung, colon, rectum, stomach, kidney, prostate, breast, pharynx, larynx, ovary, oesophagus and bladder cancer.

6. A method of diagnosing cancer in a patient, comprising:
detecting or measuring IL-6 and histone isoform H3.1, in a body fluid sample obtained from the patient; and
using the level or concentration of IL-6 and histone isoform H3.1 detected in the body fluid sample to determine if the patient has cancer.

7. The method as defined in claim 6, which additionally comprises determining at least one clinical parameter for the patient, such as age, sex and body mass index (BMI).

8. The method as defined in claim 6 or claim 7, which additionally comprises measuring IL-8 and/or IL-10.

9. The method as defined in any one of claims 6 to 8, wherein the level or concentration of IL-6 and histone isoform H3.1 detected is compared to a control.

10. The method as defined in any one of claims 6 to 9, wherein the body fluid sample is a blood, serum or plasma sample.

11. The method as defined in any one of claims 6 to 10, wherein the detecting or measuring is performed using an immunoassay or mass spectrometry.

12. The method as defined in any one of claims 6 to 11, which additionally comprises isolating the DNA associated with histone isoform H3.1 detected in the body fluid sample and optionally sequencing the DNA.

13. A kit for detection and/or quantification of IL-6 and histone isoform H3.1 in a body fluid sample comprising one or more ligands capable of specific binding to IL-6 and histone isoform H3.1.

14. The kit as defined in claim 13, for use in detecting cancer.

15. A method for assessing the suitability of a patient for a colonoscopy, comprising:
(i) detecting or measuring the level of fecal haemoglobin in a fecal sample obtained from the patient;
(ii) detecting or measuring the level of IL-6, and the level of histone isoform H3.1, in a body fluid sample obtained from the patient; and
(iii) using the measured fecal haemoglobin, IL-6 and histone isoform H3.1 level, as an indicator of the suitability of the patient for colonoscopy.

## Patentansprüche

1. *In-vitro-*Verwendung einer Palette von Biomarkern in einer Körperflüssigkeitsprobe zum Diagnostizieren und/oder Überwachen einer Krebserkrankung, wobei die Biomarker Histonisoform H3.1 und Interleukin-6 (IL-6) umfassen.

2. *In-vitro*-Verwendung nach Anspruch 1, wobei die Palette von Biomarkern zusätzlich Interleukin-8 (IL-8) und Interleukin-10 (IL-10) umfasst.

3. *In-vitro-*Verwendung nach Anspruch 1 oder 2, wobei die Körperflüssigkeitsprobe eine Blut-, Serum- oder Plasmaprobe ist.

4. *In-vitro*-Verwendung nach einem der Ansprüche 1 bis 3, wobei die Histonisoform H3.1 und IL-6 durch einen Immunoassay oder Massenspektroskopie gemessen werden.

5. *In-vitro*-Verwendung nach einem der Ansprüche 1 bis 4, wobei die Krebserkrankung ausgewählt ist aus: Lungen-, Kolon-, Rektum-, Magen-, Nieren-, Prostata-, Brust-, Rachen-, Kehlkopf-, Eierstock-, Speiseröhren- und Blasenkrebs.

6. Verfahren zum Diagnostizieren einer Krebserkrankung bei einem Patienten, umfassend:
Nachweisen oder Messen von IL-6 und Histonisoform H3.1 in einer Körperflüssigkeitsprobe, die von dem Patienten erlangt wurde; und
Feststellen, ob der Patient an einer Krebserkrankung leidet, anhand des Spiegels oder der Konzentration von IL-6 und Histonisoform H3.1, die in der Körperflüssigkeitsprobe nachgewiesen wurden.

7. Verfahren nach Anspruch 6, ferner umfassend Bestimmen mindestens eines klinischen Parameters für den Patienten, wie etwa Alter, Geschlecht und Body-Mass-Index (BMI).

8. Verfahren nach Anspruch 6 oder Anspruch 7, ferner umfassend Messen von IL-8 und/oder IL-10.

9. Verfahren nach einem der Ansprüche 6 bis 8, wobei der nachgewiesene Spiegel oder die nachgewiesene Konzentration von IL-6 und der Histonisoform H3.1 mit einer Kontrolle verglichen wird.

10. Verfahren nach einem der Ansprüche 6 bis 9, wobei die Körperflüssigkeitsprobe eine Blut-, Serum- oder Plasmaprobe ist.

11. Verfahren nach einem der Ansprüche 6 bis 10, wobei das Nachweisen oder Messen unter Verwendung eines Immunoassays oder Massenspektrometrie durchgeführt wird.

12. Verfahren nach einem der Ansprüche 6 bis 11, ferner umfassend Isolieren der DNA, die mit der in der Körperflüssigkeitsprobe nachgewiesenen Histonisoform H3.1 assoziiert ist, und optional Sequenzieren der DNA.

13. Kit zum Nachweis und/oder zur Quantifizierung von IL-6 und Histonisoform H3.1 in einer Körperflüssigkeitsprobe, umfassend einen oder mehrere Liganden, die zur spezifischen Bindung an IL-6 und Histonisoform H3.1 befähigt sind.

14. Kit nach Anspruch 13 zur Verwendung beim Nachweisen von Krebserkrankungen.

15. Verfahren zum Beurteilen der Eignung eines Patienten für eine Koloskopie, umfassend:
(i) Nachweisen oder Messen des Hämoglobinspiegels im Stuhl in einer Stuhlprobe, die von dem Patienten erlangt wurde;
(ii) Nachweisen oder Messen des Spiegels von IL-6 und des Spiegels von Histonisoform H3.1 in einer Körperflüssigkeitsprobe, die von dem Patienten erlangt wurde; und
(iii)Verwenden des gemessenen Hämoglobin-Spiegels im Stuhl, IL-6- und Histonisoform-H3.1-Spiegels als Indikator für die Eignung des Patienten für die Koloskopie.

## Revendications

1. Utilisation *in vitro* d'un panel de biomarqueurs dans un échantillon de fluide corporel pour diagnostiquer et/ou surveiller un cancer, dans laquelle les biomarqueurs comprennent l'isoforme d'histone H3.1 et l'Interleukine-6 (IL-6).

2. Utilisation *in vitro* selon la revendication 1, dans laquelle le panel de biomarqueurs comprend également de l'Interleukine-8 (IL-8) et de l'Interleukine-10 (IL-10).

3. Utilisation *in vitro* selon la revendication 1 ou la revendication 2, dans laquelle l'échantillon de fluide corporel est un échantillon de sang, de sérum ou de plasma.

4. Utilisation *in vitro* selon l'une quelconque des revendications 1 à 3, dans laquelle l'isoforme d'histone H3.1 et l'IL-6 sont mesurées par un immunoessai ou une spectroscopie de masse.

5. Utilisation *in vitro* selon l'une quelconque des revendications 1 à 4, dans laquelle le cancer est choisi parmi : le cancer du poumon, du côlon, du rectum, de l'estomac, du rein, de la prostate, du sein, du pharynx, du larynx, de l'ovaire, de l'œsophage et de la vessie.

6. Procédé de diagnostic du cancer chez un patient, comprenant :
la détection ou la mesure de l'IL-6 et de l'isoforme d'histone H3.1, dans un échantillon de fluide corporel obtenu auprès du patient ; et
l'utilisation du niveau ou de la concentration d'IL-6 et d'isoforme d'histone H3.1 détectés dans l'échantillon de fluide corporel pour déterminer si le patient est atteint d'un cancer.

7. Procédé selon la revendication 6, qui comprend également la détermination d'au moins un paramètre clinique pour le patient, tel que l'âge, le sexe et l'indice de masse corporelle (IMC).

8. Procédé selon la revendication 6 ou la revendication 7, qui comprend également la mesure de l'IL-8 et/ou de l'IL-10.

9. Procédé selon l'une quelconque des revendications 6 à 8, dans lequel le niveau ou la concentration d'IL-6 et d'isoforme d'histone H3.1 détectés est comparé à un échantillon témoin.

10. Procédé selon l'une quelconque des revendications 6 à 9, dans lequel l'échantillon de fluide corporel est un échantillon de sang, de sérum ou de plasma.

11. Procédé selon l'une quelconque des revendications 6 à 10, dans lequel la détection ou la mesure est effectuée en utilisant un immunoessai ou une spectrométrie de masse.

12. Procédé selon l'une quelconque des revendications 6 à 11, qui comprend également l'isolement de l'ADN associé à l'isoforme d'histone H3.1 détecté dans l'échantillon de fluide corporel et éventuellement le séquençage de l'ADN.

13. Kit de détection et/ou de quantification d'IL-6 et d'isoforme d'histone H3.1 dans un échantillon de fluide corporel comprenant un ou plusieurs ligands capables de se lier spécifiquement à l'IL-6 et à l'isoforme d'histone H3.1.

14. Kit selon la revendication 13, destiné à être utilisé dans la détection du cancer.

15. Procédé d'évaluation de l'aptitude d'un patient à subir une coloscopie, comprenant :
(i) la détection ou la mesure du taux d'hémoglobine fécale dans un échantillon fécal obtenu auprès du patient ;
(ii) la détection ou la mesure du niveau d'IL-6 et du niveau d'histone isoforme H3.1, dans un échantillon de fluide corporel obtenu auprès du patient ; et
(iii) l'utilisation du taux d'hémoglobine fécale mesuré, d'IL-6 et d'isoforme d'histone H3.1, comme indicateur de l'aptitude du patient à subir une coloscopie.
